(19) Europäisches Patentamt
European Patent Office
Office européen des brevets

(11) Veröffentlichungsnummer: **0 415 883 A2**

(12) **EUROPÄISCHE PATENTANMELDUNG**

(21) Anmeldenummer: **90810624.8**

(22) Anmeldetag: **20.08.90**

(51) Int. Cl.[5]: **G03C 7/392**, G03C 7/388, //C07C39/00

(30) Priorität: **28.08.89 CH 3104/89**

(43) Veröffentlichungstag der Anmeldung:
**06.03.91 Patentblatt 91/10**

(84) Benannte Vertragsstaaten:
**DE FR GB IT**

(71) Anmelder: **CIBA-GEIGY AG**
**Klybeckstrasse 141**
**CH-4002 Basel(CH)**

(72) Erfinder: **Leppard, David G., Dr.**
**Route de Bourguillon 6**
**CH-1723 Marly(CH)**
Erfinder: **Rody, Jean, Dr.**
**Rütiring 82**
**CH-4125 Riehen(CH)**

(54) **Stabilisatoren für farbphotographische Aufzeichnungsmaterialien.**

(57) Farbphotographisches Aufzeichnungsmaterial, enthaltend in mindestens einer der chromogenen farbphotographischen Schichten als Stabilisator mindestens eine Verbindung der Formel I,

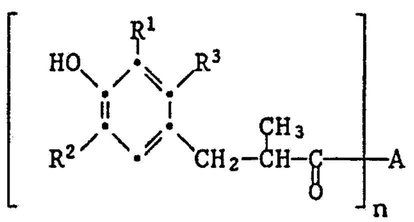

(I),

wobei
$R^1$ beispielsweise Alkyl, Cycloalkyl, mit Alkylgruppen substituiertes Cycloalkyl, Phenyl oder Aralkyl,
$R^2$ beispielsweise -H, Alkyl, Cycloalkyl mit Alkylgruppen substituiertes Cycloalkyl, Phenyl oder Aralkyl und
$R^3$ = -H oder $CH_3$ bedeuten und
n die Zahl 1 oder 2 bedeutet, wobei,
wenn n = 1 ist,
A = $-OR^4$ oder

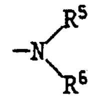

ist, und
$R^4$ beispielsweise die Bedeutung von Alkyl oder durch -O-, -S- oder $-NR^7$-unterbrochenes und gegebenenfalls durch -OH substituiertes $C_3$-$C_{20}$-Alkyl, hat, oder eine Gruppe der Formel

EP 0 415 883 A2

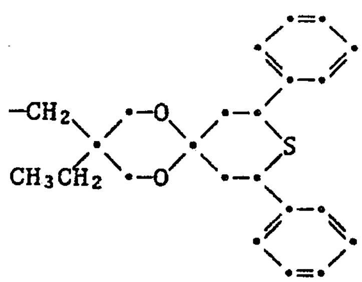

oder

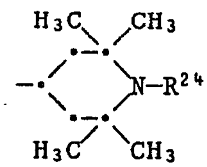

ist,
und $R^{24}$ Alkyl mit 1 bis 4 Kohlenstoffatomen, Allyl, Benzyl ein Rest der Formel

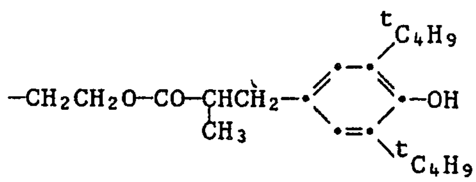

oder $COR^{25}$

ist,
worin $R^{25}$ Alkyl mit 1 bis 24 Kohlenstoffatomen oder Allyl ist,
$R^4$ die Bedeutung von durch -OH, $-OCOR^8$ oder $-P(O)(OR^9)_2$ substituiertem Alkyl hat, oder
$R^4$ beispielsweise die Bedeutung von Cycloalkyl, mit Alkylgruppen substituiertes Cycloalkyl, Alkenyl, Aralkyl, Alkaryl, Phenyl, einer Gruppe $-CH_2CH(OH)R^{10}$, wobei $R^{10}$ z.B. Aralkyl bedeutet, hat, oder
$R^4$ die Bedeutung einer Gruppe der Formel

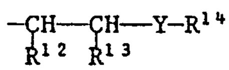

hat, wobei
$R^{12}$ und $R^{13}$, unabhängig voneinander, Wasserstoff oder Alkyl,
Y -O- oder -S- und
$R^{14}$ beispielsweise eine Gruppe der Formel

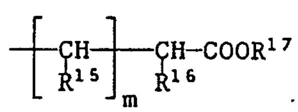

darstellen, wobei
$R^{15}$ und $R^{16}$, unabhängig voneinander, Wasserstoff oder Methyl, m 0 oder 1 und
$R^{17}$ beispielsweise Wasserstoff, Alkyl, Cycloalkyl, Alkenyl, Aralkyl, Phenyl oder Alkaryl darstellen,
$R^5$ und $R^6$, unabhängig voneinander, beispielsweise Wasserstoff, Alkyl, durch -O- unterbrochenes Alkyl, Cycloalkyl, mit Alkylgruppen substituiertes Cycloalkyl, Phenyl, durch Alkyl, Alkoxy oder Halogen substituiertes Phenyl, Alkenyl, Aralkyl oder Hydroxyalkyl darstellen, oder $R^5$ und $R^6$ zusammen Tetramethylen oder Pentamethylen oder durch -O-, $-NR^{21}$-oder $-S(O)_q$- unterbrochenes Tetramethylen oder Pentamethylen darstellen, wobei $R^{21}$ z.B. Wasserstoff, Alkyl oder Phenyl bedeutet und
$q = 0$, 1 oder 2 ist, oder,
wenn $n = 2$ ist,
A eine Gruppe der Formel $-O-X'-O-$ bedeutet, wobei
$X'$ beispielsweise Alkylen, Alkenylen, Alkinylen, Cyclohexylen, oder
A eine Gruppe der Formel

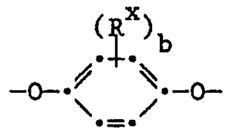

bedeutet,
wobei $R^x$ eine Alkylgruppe mit 1 bis 8 C-Atomen darstellt und b = 0, 1 oder 2 ist, oder
A eine Gruppe der Formel

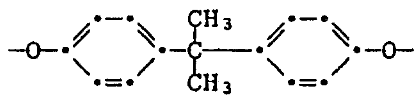

bedeutet, oder
A eine Gruppe der Formel

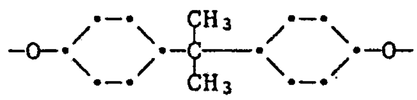

bedeutet, oder
A eine Gruppe der Formel
$-O-CH_2-CH(OH)-CH_2-O-X^2-O-CH_2-CH(OH)-CH_2-O-$ bedeutet,
wobei $X^2 = X'$ entspricht oder

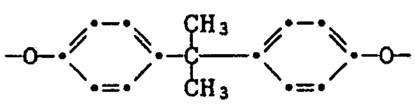

oder

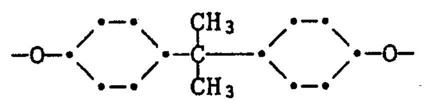

ist, oder
A eine Gruppe der Formel

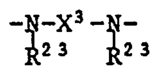 ,

wobei
$X^3$ z.B. Alkylen und $R^{23}$ Wasserstoff, Alkyl oder Phenyl sind, bedeutet.
Ein Teil der beschriebenen Verbindungen sind neu.

## STABILISATOREN FÜR FARBPHOTOGRAPHISCHE AUFZEICHNUNGSMATERIALIEN

Die vorliegende Erfindung betrifft die Stabilisierung von photographischen Farbstoffen und Farbstoffkupplern durch Zusatz eines Stabilisators in mindestens einer Schicht von photographischen Aufzeichnungsmaterialien. Der Zusatz kann z.B. in der Gelbschicht, Magentaschicht oder Cyanschicht erfolgen.

Die meisten heute gebräuchlichen Silber-Farbbild-Verfahren beruhen auf einer chemischen Reaktion zwischen einem Farbstoffkuppler - einer Verbindung mit einer reaktiven Methylen- oder Pseudomethylengruppe - und einem oxidierten Entwickler. Der Entwickler - üblicherweise ein p-Phenylendiaminderivat - wird dabei vom photosensibilisierten Silberhalogenid oxidiert unter Bildung von Silbermetall. Der oxidierte Entwickler ist das eigentliche Reagens zur Farbstoffbildung.

Entsprechend ihren chemischen Strukturen benötigen die Farbkuppler entweder 2 oder 4 Mol Silber pro Mol Kuppler. Man spricht daher von diäquivalenten oder tetraäquivalenten Kupplern.

Farbphotographische Materialien, die auf einem solchen Prozess beruhen, haben mindestens drei farbempfindliche Schichten, die drei verschiedene Sorten von Kupplern enthalten:

(a) Die Gelbschicht, enthaltend einen Gelbkuppler. Dies sind meist $\beta$-Ketocarbonamide.

(b) Die Cyanschicht, enthaltend einen Cyankuppler. Dies sind meist Phenole, Naphthole oder Pyrazolazole.

(c) Die Purpur- oder Magentaschicht, enthaltend einen Magentakuppler. Diese sind meist Pyrazolone, Pyrazoloazole, Cyanoacetylcumarone oder offenkettige Acylacetonitrile.

Um eine Diffusion der Kuppler in die Nachbarschicht zu vermeiden, versieht man die Verbindungen mit hydrophoben Ballastgruppen oder bindet sie in polymere Strukturen ein.

Die nach der Entwicklung entstandenen gelben Farbstoffe besitzen eine ungenügende Lichtechtheit. Die Lichtechtheit lässt sich zu einem gewissen Grade dadurch verbessern, dass man oberhalb der Gelbschicht eine Schicht einführt, die einen UV-Absorber enthält, wie es z.B. in der US-A-3,253,921 vorgeschlagen wurde.

Ein anderer, besserer Weg ist es, der Gelbschicht spezielle Stabilisatoren zuzusetzen. Diese dürfen nicht mit dem Kuppler oder dem Entwickler reagieren. Als solche Stabilisatoren wurden verschiedene phenolische Verbindungen vorgeschlagen, insbesondere aber p-Hydroxybenzoesäureester-Derivate, wie sie z.B. in der US-A-4,228,235 vorgeschlagen wurden, und Kombinationen von sterisch gehinderten Aminen mit Phenolen, wie sie z.B. in der EP-A-114,029 vorgeschlagen wurden. Auch molekulare Kombinationen von gehinderten Aminen und phenolischen Gruppen wurden vorgeschlagen, z.B. in der EP-A-82817 und EP-A-103,540. Solche Stabilisatoren haben zu einer erheblichen Steigerung der Lichtechtheit geführt, aber man ist weiterhin an Verbesserungen der Lichtechtheit der Gelbfarbstoffe interessiert.

Auch die Magenta-Farbstoffe und -Kuppler sind nicht genügend lichtecht. Dazu kommt eine thermische Instabilität vieler Magenta-Kuppler bei der Dunkellagerung. Wie bei den Gelbfarbstoffen hat man auch für die Magenta-Farbstoffe und -Kuppler Filterschichten mit UV-Absorbern, insbesondere mit Hydroxyphenylbenztriazolen vorgeschlagen, z.B. in der US-A-3,533,794, jedoch genügt dies nicht für die heutigen Anforderungen an Lichtechtheit.

Wirksam ist der Zusatz von Stabilisatoren in die Magenta-Schicht. Als solche eignen sich insbesondere Hydrochinonderivate, wie sie z.B. in der US-A-3,935,016 beschrieben sind.

Auch die Cyan-Farbstoffe müssen stabilisiert werden. Dies kann ebenfalls durch UV-Absorber-Filterschichten geschehen oder durch Zusatz von speziellen Stabilisatoren zur Cyanschicht. Solche Stabilisatoren können z.B. Phenole oder gehinderte Amine sein, wie in der EP-A-113,124 beschrieben.

Es sind auch aus der JP-A 49-134 326 photographische Farbfilme bekannt, enthaltend Stabilisatoren der Formel

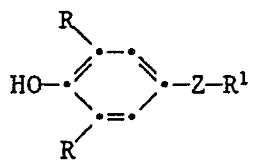

oder

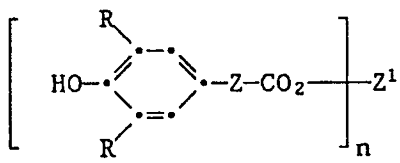

,

wobei R = tert. $C_4$- oder $C_5$-Alkyl, Z = 1-3 C-Alkylen oder Imino, $R^1$ = C-alkylthiodisubstituiertes s-Triazin und $Z^1$ eine polyvalente aliphatische oder cycloaliphatische Gruppe bedeuten.

Aus der JP-A 59-005-246 sind farbphotographische Produkte bekannt, die auf einem Träger eine Schicht eines Gelbfarbstoffes aufweisen und im Gelbfarbstoff eine Verbindung der Formel

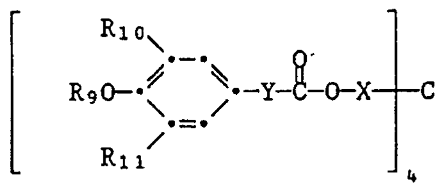

,

wobei $R_9$ = -H, Alkyl oder Aryl, $R_{10}$ und $R_{11}$ Alkyl, X Alkylen oder Arylen und Y Alkylen ist.

Aehnliche wie obengenannte Verbindungen sind überdies aus den JP-A 50-023 822 und 54-154 325, sowie aus EP-A 310551, 310552 und 309917 bekannt geworden.

Die genannten Verbindungen konnten jedoch nicht in allen Belangen, beispielsweise beim Lichtschutz, überzeugen. Es war daher Aufgabe vorliegender Erfindung neue Stabilisatoren zur Stabilisierung von farbphotographischen Materialien zur Verfügung zu stellen. Ein Teil der als Stabilisatoren genannten Verbindungen sind neu.

Gegenstand vorliegender Erfindung ist ein farbphotographisches Aufzeichnungsmaterial, enthaltend in mindestens einer der chromogenen farbphotographischen Schichten als Stabilisator mindestens eine Verbindung der Formel I,

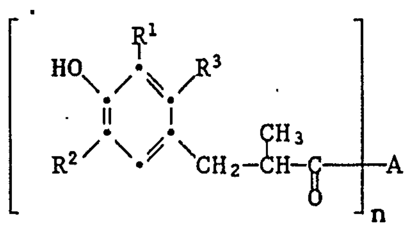

(I),

wobei

$R^1$ = Alkyl mit 1 bis 18 C-Atomen, Cycloalkyl mit 5 bis 12 C-Atomen, mit 1 bis 3 $C_1$-$C_4$-Alkylgruppen substituiertes Cycloalkyl mit 5 bis 12 Ring-C-Atomen, Phenyl oder Aralkyl mit 7 bis 9 C-Atomen,

$R^2$ = -H, Alkyl mit 1 bis 18 C-Atomen, Cycloalkyl mit 5 bis 12 C-Atomen, mit 1 bis 3 $C_1$-$C_4$-Alkylgruppen substituiertes Cycloalkyl mit 5 bis 12 Ring-C-Atomen, Phenyl oder Aralkyl mit 7 bis 9 C-Atomen und

$R^3$ = -H oder $CH_3$ bedeutet und

n die Zahl 1 oder 2 bedeutet, wobei,

wenn n = 1 ist,

A = $-OR^4$ oder

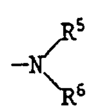

ist, und

$R^4$ die Bedeutung von Alkyl mit 1 bis 24 C-Atomen, durch wenigstens ein -O-, -S- oder $-NR^7-$ unterbrochenes $C_3$-$C_{20}$-Alkyl oder durch wenigstens ein -O-, -S- oder $-NR^7-$ unterbrochenes $C_3$-$C_{20}$-Alkyl, das mit wenigstens einer Hydroxygruppe substituiert ist, hat, oder eine Gruppe der Formel

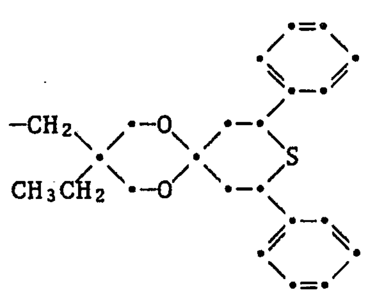

oder

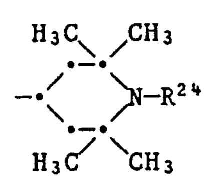

ist,
wobei
$R^7$ Wasserstoff, $C_1$-$C_{12}$-Alkyl oder Phenyl und $R^{24}$ Alkyl mit 1 bis 4 Kohlenstoffatomen, Allyl, Benzyl ein Rest der Formel

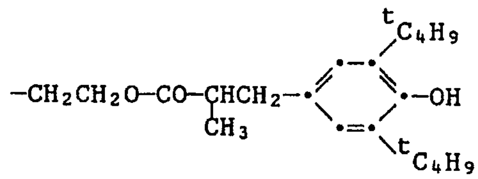

oder $COR^{25}$ ist,
worin $R^{25}$ Alkyl mit 1 bis 24 Kohlenstoffatomen oder Allyl ist, oder $R^4$ die Bedeutung von durch -OH, $-OCOR^8$ oder $-P(O)(OR^9)_2$ substituiertem $C_1$-$C_{24}$-Alkyl hat, wobei
$R^8$ $C_1$-$C_{10}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl, oder $C_7$-$C_{15}$-Alkaryl und
$R^9$ Wasserstoff, $C_{1-18}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl, Naphthyl oder $C_7$-$C_{15}$-Alkaryl bedeutet, hat, oder
$R^4$ die Bedeutung von $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, $C_7$-$C_{18}$-Alkaryl, Phenyl, einer Gruppe $-CH_2CH(OH)R^{10}$, wobei $R^{10}$ $C_7$-$C_{12}$-Aralkyl oder $-CH_2OR^{11}$ und $R^{11}$ Cyclohexyl, Phenyl, Tolyl oder Benzyl bedeutet, hat, oder
$R^4$ die Bedeutung einer Gruppe der Formel

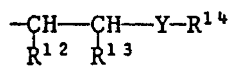

hat, wobei
$R^{12}$ und $R^{13}$ unabhängig voneinander, Wasserstoff oder $C_1$-$C_{18}$-Alkyl,
Y -O- oder -S- und
$R^{14}$ eine Gruppe der Formel

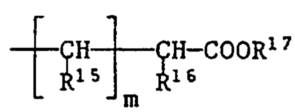

darstellt, wobei
$R^{15}$ und $R^{16}$, unabhängig voneinander, Wasserstoff oder Methyl, m 0 oder 1 und
$R^{17}$ Wasserstoff, $C_1$-$C_{24}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl oder $C_7$-$C_{16}$-Alkaryl darstellt, oder
$R^{14}$ eine Gruppe der Formel

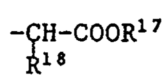

darstellt, wobei $R^{18}$ Wasserstoff, $C_1$-$C_{18}$-Alkyl oder eine Gruppe $COOR^{17}$, wobei $R^{17}$ genannte Bedeutung hat, darstellt, oder
$R^{14}$ Wasserstoff, Phenyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_8$Alkenyl, Benzyl oder eine Gruppe der Formel

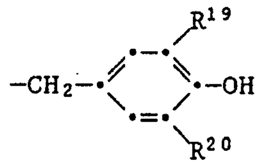

darstellt, wobei

$R^{19}$ und $R^{20}$, unabhängig voneinander -H, Methyl oder $-C(CH_3)_3$ bedeuten, mit der Massgabe, dass $R^{19}$ und $R^{20}$ nicht gleichzeitig -H sind, und $R^5$ und $R^6$, unabhängig voneinander, -H, $C_1$-$C_{12}$-Alkyl, durch ein oder mehrere -O- unterbrochenes $C_3$-$C_{20}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, Phenyl, durch $C_1$-$C_{12}$-Alkyl, $C_1$-$C_4$-Alkoxy oder Halogen substituiertes Phenyl, $C_3$-$C_8$-Alkenyl, $C_7$-$C_{15}$-Aralkyl, $C_2$-$C_4$-Hydroxyalkyl darstellen, oder

$R^5$ und $R^6$ zusammen stellen Tetramethylen oder Pentamethylen oder durch -O-, $-NR^{21}$-oder $-S(O)_q$ -unterbrochenes Tetramethylen oder Pentamethylen dar, wobei $R^{21}$ Wasserstoff, $C_1$-$C_{12}$-Alkyl oder Phenyl und q = 0, 1 oder 2 ist, oder,

wenn n = 2 ist,

A eine Gruppe der Formel $-O-X'-O-$ bedeutet, wobei

$X'$ = $C_2$-$C_{10}$-Alkylen, $C_4$-$C_8$-Alkenylen, $C_4$-$C_8$-Alkinylen, Cyclohexylen, durch ein oder mehrere Glieder der Reihe

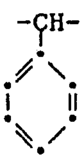 ,

-O-, -S(O)q und $-NR^{22}$-unterbrochenes $C_4$-$C_{40}$-Alkylen, wobei $R^{22}$ Wasserstoff oder $C_3$-$C_{12}$-Alkyl oder Phenyl ist, und q = 0, 1 oder 2 bedeutet, oder

A eine Gruppe der Formel

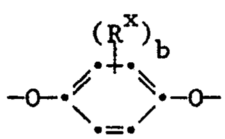

bedeutet,

wobei $R^x$ eine Alkylgruppe mit 1 bis 8 C-Atomen darstellt und b = 0, 1 oder 2 ist, oder

A eine Gruppe der Formel

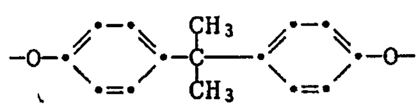

bedeutet, oder

A eine Gruppe der Formel

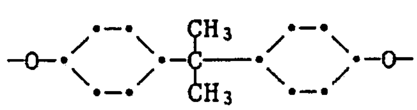

bedeutet, oder

A eine Gruppe der Formel

$-O-CH_2-CH(OH)-CH_2-O-X^2-O-CH_2-CH(OH)-CH_2-O-$ bedeutet,

wobei $X^2$ = $X'$ entspricht oder

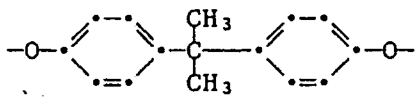 oder 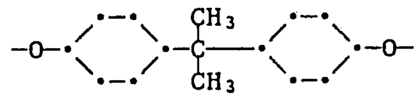

ist, oder
A eine Gruppe der Formel

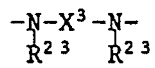 ,

wobei
$X^3$ $C_2$-$C_{10}$-Alkylen und $R^{23}$ Wasserstoff, $C_1$-$C_{12}$-Alkyl oder Phenyl ist, bedeutet.

Zweckmässig ist ein farbphotographisches Aufzeichnungsmaterial, wie oben beschrieben, enthaltend mindestens eine Verbindung der Formel I, wobei $R^1$ = Alkyl mit 1 bis 8 C-Atomen, Cyclohexyl, Phenyl oder $C_7$-$C_9$-Phenylalkyl,
$R^2$ = Wasserstoff, Alkyl mit 1 bis 8 C-Atomen, Cyclohexyl, Phenyl oder $C_7$-$C_9$-Phenylalkyl, und
$R^3$ = Wasserstoff bedeuten.

Ein anderes zweckmässiges farbphotographisches Aufzeichnungsmaterial enthält mindestens eine Verbindung der Formel I, wobei, wenn n = 1 ist, A die Bedeutung von $-OR^4$ oder $-NR^5R^6$ hat, wobei
$R^4$ die Bedeutung von Alkyl mit 1 bis 18 C-Atomen, durch 1 bis 3 -O- oder -S- unterbrochenes $C_3$-$C_{20}$-Alkyl oder durch 1 bis 3 -O- oder -S- unterbrochenes $C_3$-$C_{20}$-Alkyl, das mit 1 bis 2 -OH substituiert ist, oder
$R^4$ die Bedeutung von durch $-P(O)(OR^9)_2$ substituierten $C_1$-$C_9$-Alkyl hat, wobei $R^9$ Wasserstoff, $C_1$-$C_{12}$-Alkyl, $C_5$-$C_{12}$-Cycloalkyl, $C_3$-$C_{18}$-Alkenyl, Benzyl, Phenyl oder Tolyl bedeutet, oder
$R^4$ die Bedeutung von $C_5$-$C_{12}$-Cycloalkyl, $C_2$-$C_{18}$-Alkenyl, $C_7$-$C_{10}$-Aralkyl, $C_7$-$C_{18}$-Aralkyl, Phenyl oder einer Gruppe $-CH_2CH(OH)R^{10}$ hat, wobei
$R^{10}$ $C_7$-$C_{10}$-Aralkyl oder $-CH_2OR^{11}$ bedeutet, mit der Bedeutung für $R^{11}$ von Cyclohexyl, Phenyl, Tolyl oder Benzyl, oder
$R^4$ eine Gruppe der Formel

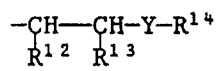

bedeutet, wobei
$R^{12}$ und $R^{13}$, unabhängig voneinander, Wasserstoff oder $C_1$-$C_{12}$-Alkyl bedeuten,
Y = -O- oder -S- ist, und
$R^{14}$ eine Gruppe der Formel

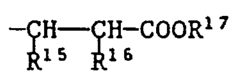

ist,
wobei
$R^{15}$ und $R^{16}$, unabhängig voneinander, Wasserstoff oder Methyl und
$R^{17}$ $C_1$-$C_{12}$-Alkyl, Cyclohexyl, $C_2$-$C_{18}$-Alkenyl, $C_7$-$C_9$-Aralkyl, Phenyl oder $C_7$-$C_{16}$-Alkaryl sind, oder
$R^{14}$ eine Gruppe der Formel

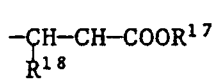

ist, wobei
$R^{18}$ Wasserstoff, $C_1$-$C_{12}$-Alkyl oder eine Gruppe $COOR^{17}$ darstellt und wobei
$R^{17}$ die obengenannte Bedeutung hat, oder
$R^{14}$ Phenyl, Cyclohexyl, $C_2$-$C_9$-Alkenyl, Benzyl oder eine Gruppe der Formel

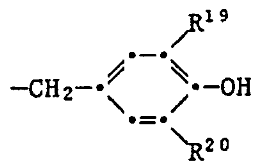

darstellt,
wobei
$R^{19}$ und $R^{20}$, unabhängig voneinander, -H, Methyl oder $-C(CH_3)_3$ darstellen, mit der Massgabe, dass $R^{19}$ und $R^{20}$ nicht gleichzeitig -H sind, und
$R^5$ und $R^6$, unabhängig voneinander, Wasserstoff, $C_1$-$C_{12}$-Alkyl, durch 1 bis 3 -O- unterbrochenes $C_3$-$C_{20}$-Alkyl, Cyclohexyl, Phenyl, $C_3$-$C_6$-Alkenyl oder Benzyl, oder
$R^5$ und $R^6$ zusammen Tetramethylen oder Pentamethylen oder durch ein -O-unterbrochenes Tetramethylen darstellen, oder,
wenn n = 2 ist,
A eine Gruppe der Formel -O-X′-O- darstellt, wobei
X′ die Bedeutung $C_2$-$C_8$-Alkylen, $C_4$-$C_8$-Alkenylen, $C_4$-$C_{12}$-Alkylen, das durch 1 bis 3 Glieder der Reihe

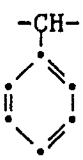,

-O-, -S(O)q und $-NR^{22}-$ unterbrochen ist,
wobei $R^{22}$ die Bedeutung von Wasserstoff, $C_1$-$C_{12}$-Alkyl oder Phenyl hat, Phenylen, $-CH_2C{\equiv}C-CH_2-$ oder $(CH_2CH_2O)_aCH_2CH_2-$, wobei a = 1, 2 oder 3 ist und q = 0,1 oder 2 bedeutet, hat, oder A die Bedeutung einer Gruppe der Formel

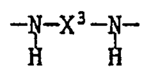

hat, wobei $X^3$ Alkylen mit 4 bis 8 C-Atomen ist.

Bevorzugt ist ein farbphotographisches Aufzeichnungsmaterial, enthaltend mindestens eine Verbindung der Formel I, wobei $R^1$ Alkyl mit 1 bis 4 C-Atomen, $R^2$ Wasserstoff oder Alkyl mit 1 bis 4 C-Atomen und $R^3$ Wasserstoff bedeuten.

Ganz besonders bevorzugt in Verbindungen der Formel I ist für $R^1$ und $R^2$ tert.-Butyl und für $R^3$ Wasserstoff.

Ein anderes bevorzugtes farbphotographisches Aufzeichnungsmaterial enthält mindestens eine Verbindung der Formel I, wobei,
wenn n = 1 ist,
A die Bedeutung von $-OR^4$ hat, wobei
$R^4$ Alkyl mit 1 bis 18 C-Atomen, Alkenyl mit 2 bis 18 C-Atomen oder durch ein oder zwei -O-unterbrochenes Alkyl mit 3 bis 12 C-Atomen bedeutet, oder $R^4$ eine Gruppe der Formel $CH_2CH_2$-S-$(CH_2)_m$-$CH_2$-$COOR^{17}$ bedeutet, wobei
m 0 oder 1 und $R^{17}$ $C_1$-$C_{12}$-Alkyl sind, oder
$R^4$ $-CH_2-P(O)OR^9)_2$ oder $-CH_2-CH_2-P(O)(OR^9)_2$ bedeutet, wobei
$R^9$ $C_2$-$C_5$-Alkyl ist, oder
A die Bedeutung von

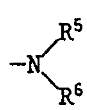

hat,

wobei
$R^5$ = Wasserstoff ist und
$R^6$ = $C_2$-$C_4$-Alkenyl oder durch -O- unterbrochenes $C_3$-$C_8$-Alkyl ist, oder
wenn n = 2 ist,
A die Bedeutung einer Gruppe der Formel -O-X′-O- hat, wobei
X′ eine Alkylengruppe mit 2 bis 4 C-Atomen, $-CH_2-CH=CH-CH_2-$, eine Alkenylengruppe mit 4 bis 8 C-Atomen oder eine durch ein -S-, -O-, oder

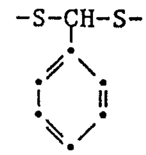

unterbrochene Alkylengruppe mit 2 bis 6 C-Atomen ist, oder
A die Bedeutung einer Gruppe der Formel

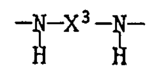

hat, wobei $X^3$ Alkylen mit 4 bis 8 C-Atomen ist.

Besonders bevorzugt ist ein farbphotographisches Aufzeichnungsmaterial, enthaltend mindestens eine Verbindung der Formel I, worin $R^1$ und $R^2$ eine tert.-Butylgruppe und $R^3$ Wasserstoff bedeuten.

Besonders bevorzugt ist auch ein farbphotographisches Aufzeichnungsmaterial enthaltend mindestens eine Verbindung der Formel I, worin,
wenn n = 1 ist,
A die Bedeutung von $-OR^4$ hat, wobei
$R^4$ Alkyl mit 1-18 C-Atomen, Alkenyl mit 12 bis 18 C-Atomen, $-CH_2-CH_2-S-C(CH_3)_3$ oder $-CH_2-CH_2-O-CH_2-CH_2-O-C_2H_5$ bedeutet, oder,
wenn n = 2 ist,
A die Bedeutung von -O-X′-O- hat, wobei X′ Alkylen mit 2 bis 4 C-Atomen oder durch ein -O- oder -S- unterbrochenes Alkylen mit 4 bis 6 C-Atomen ist.

Weitere besonders bevorzugte farbphotographische Aufzeichnungsmaterialien enthalten mindestens eine Verbindung der Formel I, worin $R^1$ und $R^2$ jeweils tert.-Butyl und
$R^3$ Wasserstoff bedeuten, und, wenn n = 1 ist, A $-OR^4$ ist, wobei $R^4$ die Bedeutung von $-CH_3$,

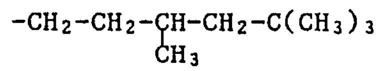

oder $C_{18}H_{37}$ hat oder,
wenn n = 2 ist,
A die Bedeutung von -O-X′-O- hat, wobei X′ = $-CH_2-CH_2-S-CH_2-CH_2-$ oder $-CH_2-CH_2-O-CH_2-CH_2-$ ist.

Anderes besonders, bevorzugtes farbfotographisches Aufzeichunungsmaterial enthält mindestens eine Verbindung der Formel I, worin $R^1$ tert.-Butyl, $R^2$ und $R^3$ Wasserstoff darstellen, und, wenn n = 1 ist, A eine Gruppe $-OR^4$ darstellt, wobei $R^4$ Methyl, $-C_8H_{17}$ oder $-(CH_2)_8CH=CH(CH_2)_7CH_3$ ist oder, wenn n = 2 ist, A eine Gruppe der Formel -O-X′-O- darstellt und dabei X′ $-CH_2-CH_2-S-CH_2-CH_2-$ oder $-CH_2-CH_2-O-CH_2-CH_2-$ ist.

Bedeuten $R^1$, $R^2$, $R^8$, $R^9$, $R^{12}$, $R^{13}$ oder $R^{18}$ beispielsweise Alkyl mit 1 bis 18 C-Atomen, so sind damit z.B. Methyl, Ethyl, Propyl, Isopropyl, n-Butyl, Isobutyl, t-Butyl, Pentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1,1,3,3-Tetramethylhexyl, 1-Methylundecyl oder 3,5,5-Trimethylhexyl-umfasst.

$R^4$ oder $R^{17}$ können Alkyl mit 1 bis 24 C-Atomen bedeuten. Beispiele lassen sich aus obiger Aufzählung entnehmen, ergänzend sei beispielsweise auf Eicosyl, Henicosyl und Docosyl hingewiesen.

$R^5$ $R^6$, $R^7$, $R^9$, $R^{21}$, $R^{22}$ oder $R^{23}$ können $C_1$-$C_{12}$-Alkyl bedeuten. Sinngemäss lassen sich aus der

obigen Aufzählung entsprechende Beispiele entnehmen.

R′ kann $C_1$-$C_8$-Alkyl bedeuten, wobei auch hier entsprechende Beispiele der Aufstellung zu entnehmen sind.

$R^1$, $R^2$, $R^4$, $R^5$, $R^6$, $R^9$, $R^{14}$, $R^{15}$, $R^{16}$ oder $R^{17}$ können $C_5$-$C_{12}$-Cycloalkyl darstellen. Beispiele für solche Gruppen sind Cyclopentyl, Cyclohexyl, Cyclooctyl oder Cyclododecyl, wobei Cyclohexyl besonders bevorzugt ist.

Beispiele für mit 1 bis 3 $C_1$-$C_4$-Alkylgruppen substituiertes Cycloalkyl mit 5 bis 12 Ring-C-Atomen sind 2- oder 4-Methylcyclohexyl, Dimethylcyclohexyl, Trimethylcyclohexyl oder t-Butylcyclohexyl.

Ist die Bedeutung von $R^1$, $R^2$, $R^3$, $R^5$, $R^6$, $R^8$, $R^9$, $R^{10}$, $R^{14}$ oder $R^{17}$ Aralkyl mit 7 bis 9 C-Atomen, resp. mit 7 bis 15 C-Atomen, so sind Phenylalkylgruppen bevorzugt. Beispiele dafür sind Benzyl, Phenethyl, α-Methylbenzyl oder α,α-Dimethylbenzyl. Bevorzugt ist Benzyl.

Bedeutet beispielsweise $R^4$ durch wenigstens ein -O-, -S- oder -$NR^7$ -unterbrochenes $C_3$-$C_{20}$-Alkyl, so sind dies beispielsweise Reste wie $-CH_2-CH_2-S-C(CH_3)_3$ oder $-CH_2-CH_2-O-CH_2-CH_2-O-C_2H_5$. Als Beispiel für $R^4$, als ein durch wenigstens ein -O-, -S- oder -$NR^7$- unterbrochenes $C_3$-$C_{20}$-Alkyl, das mit wenigstens einer -OH-Gruppe substituiert ist, kann $-CH_2-CH_2-O-CH_2-CH_2-O-CH_2-CH_2-OH$ angeführt werden. Beispiele für einen Rest $R^4$ mit der Bedeutung von durch $-P(O)(OR^9)_2$ substituiertem $C_1$-$C_{24}$-Alkyl sind $-CH_2-P(O)-(OC_4H_9)_2$ und $-CH_2-CH_2-P(O)(OC_4H_9)_2$.

$R^4$, $R^5$, $R^6$, $R^8$, $R^9$, $R^{14}$ $R^{15}$ oder $R^{16}$ können $C_2$-$C_{18}$-Alkenyl, resp. $C_3$-$C_8$-Alkenyl, bedeuten.
Beispiele dafür lassen sich sinngemäss aus Allyl, 2-Methallyl, 2-Butenyl, trans-2-Butenyl, 2-Hexenyl, trans-2,4-Hexadienyl, resp. Hexenyl, Decenyl, Undecenyl, Heptadecenyl, Oleyl, cis-9-Octadecenyl, trans-9-Octadecenyl, cis,cis-9,12-Octadecadienyl oder cis,cis,cis-9,12,15-Octadecatrienyl auswählen.

Bevorzugt sind cis-9-Octadecenyl und trans-9-Octadecenyl.

$R^8$ und $R^9$ können $C_7$-$C_{15}$-Alkaryl, $R^{17}$ kann $C_7$-$C_{16}$-Alkaryl und $R^4$ kann $C_7$-$C_{18}$-Alkaryl darstellen. Beispiele sind Methylphenyl, Dimethylphenyl, Trimethylphenyl, Ethylphenyl, Isopropylphenyl, t-Butylphenyl, Methyl-t-butylphenyl, Di-t-butylphenyl oder 2,6-Di-t-butyl-4-methylphenyl.

Beispiele für $R^4$ als Gruppe der Formel

$$-\underset{R^{12}}{CH}-\underset{R^{13}}{CH}-Y-R^{14}\text{, wobei } R^{14} = -\left[\underset{R^{15}}{CH}\right]_m-\underset{R^{16}}{CH}-COOR^{17}\text{ ist,}$$

sind $-CH_2-CH_2-S-CH_2-CH_2-COOC_1$-$C_8$-Alkyl und $-CH_2-CH_2-S-CH_2-COOC_1$-$C_8$-Alkyl. Die Bedeutung von $C_1$-$C_8$-Alkyl kann Methyl, Ethyl, n-Propyl, i-Propyl, n-Butyl, i-Butyl, t-Butyl, Pentyl, Hexyl, Heptyl oder Octyl sein.

$R^5$ und $R^6$ können auch durch wenigstens mindestens ein -O- unterbrochenes $C_3$-$C_{20}$-Alkyl darstellen. Beispiele dafür sind $-CH_2-CH_2-O-CH_3$ oder $-CH_2-CH_2-O-CH_2-CH_2-O-C_1$-$C_8$-Alkyl, wobei $C_1$-$C_8$-Alkyl sinngemäss obiger Aufstellung entnommen werden kann.

Bedeutet beispielsweise $R^5$ und/oder $R^6$ durch $C_1$-$C_{12}$-Alkyl, $C_1$-$C_4$-Alkoxy oder Halogen substituiertes Phenyl, so sind Beispiele dafür o-, m- oder bevorzugt p-Toluidin, 2- oder bevorzugt 4-Chloranilin oder o-, m- oder bevorzugt p-Anisidin (Methoxy-anilin).

$R^5$ und $R^6$ kann auch für $C_2$-$C_4$-Hydroxyalkyl stehen. Beispiele sind $-CH_2-CH_2-OH$, $-CH_2-CH_2-CH_2-OH$ oder $-CH_2-CH_2-CH_2-CH_2-OH$.

$R^5$ und $R^6$ zusammen können $C_4$- oder $C_5$-Alkylen, demnach Tetramethylen oder Pentamethylen oder durch -O-, $-S(O)_q$ oder -$NR^{21}$ unterbrochenes Tetramethylen- oder Pentamethylen darstellen, wobei $R^{21}$ und q die oben angegebene Bedeutung haben können. Beispiele dafür sind
$-CH_2-CH_2-O-CH_2-CH_2-$, $-CH_2-CH_2-NH-CH_2-CH_2-$ oder $-CH_2-CH_2-S(O)2-CH_2-CH_2-$.

Stellen $X^1$, $X^2$ oder $X^3$ $C_2$-$C_{10}$-Alkylen dar, so sind Beispiele dafür Ethylen, Trimethylen, Tetramethylen, Pentamethylen, Hexamethylen, Heptamethylen, Octamethylen, Decamethylen, 1-Methyl-1,2-ethandiyl, 1-Ethyl-1,2-ethandiyl, 2,2-Dimethyl-1,3-propandiyl oder 2-Ethyl-2-n-butyl-1,3-propandiyl.Beispiele für Alkylen mit 4 bis 40 C-Atomen, das durch

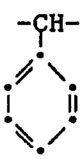 ,

-O-, -S(O)q- und/oder -$NR^{22}$, wobei $R^{22}$ genannte Bedeutungen haben kann und q = 0, 1 oder 2 bedeutet, unterbrochen ist, sind

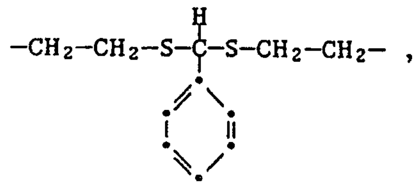

$-CH_2-CH_2-O-CH_2-CH_2-$, $-CH_2-CH_2-S-CH_2-CH_2-$oder $-CH_2-CH_2-S(O)_2-CH_2-CH_2-$.

X′ kann auch $C_4$-$C_8$-Alkenylen darstellen, wie z.B. 2-Buten-1,4-diyl.

Bedeutet X′ $C_4$-$C_8$-Alkinylen, ein Beispiele dafür ist 2-Butin-1,4-diyl.

Bevorzugt werden Verbindungen der Formel I eingesetzt, in denen A $-OR^4$ bzw. -O-X′-O- ist.

Bevorzugtes farbphotographisches Aufzeichnungsmaterial enthält in den chromogenen farbphotographischen Schichten mindestens eine Verbindung der Formel I, wie oben beschrieben. Typische Beispiele solcher Verbindungen der Formel I sind in nachfolgender Tabelle aufgelistet. $R^1$, $R^2$, $R^3$, n und A aus der Formel I haben die in untenstehender Tabelle angegebenen Bedeutungen.

Tabelle:

| $R^1$ | $R^2$ | $R^3$ | n | A |
|---|---|---|---|---|
| $C(CH_3)_3$ | $C(CH_3)_3$ | H | 1 | $-OCH_3$ |
| $C(CH_3)_3$ | $C(CH_3)_3$ | H | 1 | $-OCH_{18}H_{37}$ |
| $C(CH_3)_3$ | $C(CH_3)_3$ | H | 1 | $-OCH_2CH(C_2H_5)C_4H_9$ |
| $C(CH_3)_3$ | $C(CH_3)_3$ | H | 1 | $-OCH_2CH_2SC(CH_3)_3$ |
| $C(CH_3)_3$ | $C(CH_3)_3$ | H | 2 | $-OCH_2CH_2SCH_2CH_2O-$ |
| $C(CH_3)_3$ | $C(CH_3)_3$ | H | 1 | $-OCH_2CH_2SCH_2CH_2COOCH_3$ |
| $C(CH_3)_3$ | $C(CH_3)_3$ | H | 1 | $-OCH_2CH_2SCH_2COOC_2H_5$ |
| $C(CH_3)_3$ | $C(CH_3)_3$ | H | 2 | 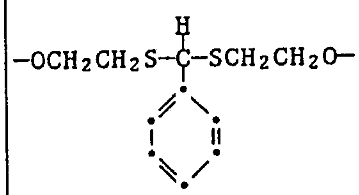 |
| $C(CH_3)_3$ | $C(CH_3)_3$ | H | 1 | $-NHCH_2CH=CH_2$ |
| $C(CH_3)_3$ | $C(CH_3)_3$ | H | 1 | $-NHCH_2CH_2OCH_3$ |
| $C(CH_3)_3$ | $C(CH_3)_3$ | H | 1 | $-OCH_2CH_2OCH_2CH_2OC_2H_5$ |
| $C(CH_3)_3$ | $C(CH_3)_3$ | H | 1 | $-OCH_2P(O)(OC_4H_9)_2$ |
| $C(CH_3)_3$ | $C(CH_3)_3$ | H | 2 | $-OCH_2CH_2OCH_2CH_2O-$ |
| $C(CH_3)_3$ | $C(CH_3)_3$ | H | 2 | $-OCH_2CH=CHCH_2O-$ |
| $C(CH_3)_3$ | $C(CH_3)_3$ | H | 2 | $-OCH_2C\equiv CCH_2O-$ |
| $C(CH_3)_3$ | $C(CH_3)_3$ | H | 2 | $-NH(CH_2)_6NH-$ |
| $C(CH_3)_3$ | $C(CH_3)_3$ | H | 2 | $-OCH_2CH_2O-$ |
| $C(CH_3)_3$ | H | H | 1 | $-OCH_3$ |
| $C(CH_3)_3$ | H | H | 1 | $-OC_3H_{17}$ |
| $C(CH_3)_3$ | H | H | 1 | $-O(CH_2)_8CH=CH(CH_2)_7CH_3$ |
| $C(CH_3)_3$ | H | H | 2 | $-OCH_2CH_2SCH_2CH_2O$ |

Ein Teil der Verbindungen, welche als Stabilisatoren in den chromogenen farbphotographischen Schichten gemäss vorliegender Erfindung beschrieben werden, sind neu.

Die neuen Verbindungen gemäss vorliegender Erfindung weisen die allgemeine Formel Ia auf,

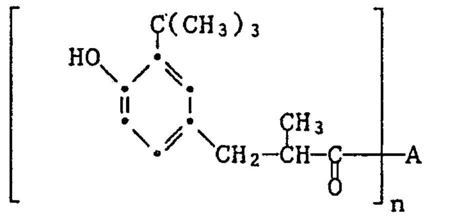 ,  Ia

worin n eine Zahl von 1 oder 2 bedeutet und, wenn n = 1 ist, A = $-OR^4$ oder

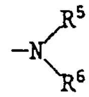

ist, und $R^4$ die Bedeutung von -H, Alkyl mit 1 bis 24 C-Atomen, durch wenigstens ein -O-, -S- oder $-NR^7-$ unterbrochenes $C_3$-$C_{20}$-Alkyl oder durch wenigstens ein -O-, -S- oder $-NR^7-$ unterbrochenes $C_3$-$C_{20}$-Alkyl, das mit wenigstens einer Hydroxygruppe substituiert ist, hat, oder eine Gruppe der Formel

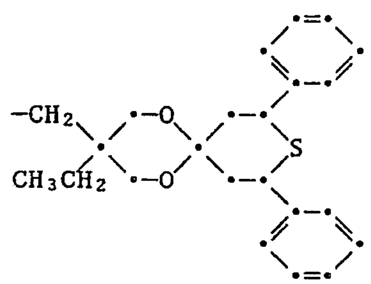 oder 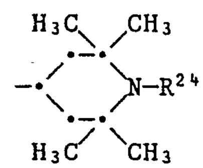 ist,

wobei
$R^7$ Wasserstoff, $C_1$-$C_{12}$-Alkyl oder Phenyl und $R^{24}$ Alkyl mit 1 bis 4 Kohlenstoffatomen, Allyl, Benzyl ein Rest der Formel

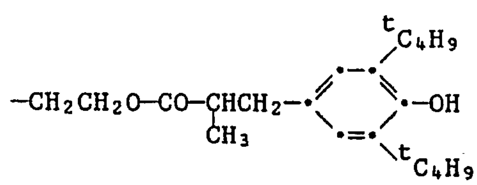

oder $COR^{25}$ ist,
worin $R^{25}$ Alkyl mit 1 bis 24 Kohlenstoffatomen oder Allyl ist, oder
$R^4$ die Bedeutung von durch -OH, $-OCOR^8$, oder $-P(O)(OR^9)_2$ substituiertem $C_1$-$C_{24}$-Alkyl hat, wobei
$R^8$ $C_1$-$C_{18}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Naphthyl, Phenyl oder $C_7$-$C_{15}$-Alkaryl und
$R^9$ Wasserstoff, $C_{1-18}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl, Naphthyl oder $C_7$-$C_{15}$-Alkaryl bedeutet, oder
$R^4$ die Bedeutung von $C_5$-$C_{12}$-Cycloalkyl, mit 1 bis 3 $C_1$-$C_4$-Alkylgruppen substituiertem Cycloalkyl mit 5 bis 12 Ring-C-Atomen, $C_2$-$C_{18}$-Alkenyl, $C_7$-$C_{15}$-Aralkyl, $C_7$-$C_{18}$-Alkaryl, Phenyl, Naphthyl, einer Gruppe $-CH_2CH(OH)R^{10}$, wobei $R^{10}$ $C_7$-$C_{12}$-Aralkyl oder $-CH_2OR^{11}$ und $R^{11}$ Cyclohexyl, Phenyl, Tolyl oder Benzyl bedeutet, hat, oder
$R^4$ die Bedeutung einer Gruppe der Formel

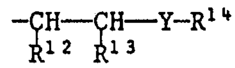

hat, wobei
$R^{12}$ und $R^{13}$, unabhängig voneinander, Wasserstoff oder $C_1$-$C_{18}$-Alkyl,
Y -O- oder -S- und
$R^{14}$ eine Gruppe der Formel

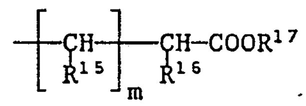

darstellt wobei,
$R^{15}$ und $R^1$, unabhängig voneinander, Wasserstoff oder Methyl, m 0 oder 1 und
$R^{17}$ Wasserstoff, $C_1$-$C_{24}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl oder $C_7$-$C_{16}$-Alkaryl darstellt, oder
$R^{14}$ eine Gruppe der Formel

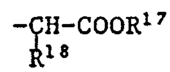

darstellt, wobei $R^{18}$ Wasserstoff, $C_1$-$C_{18}$-Alkyl oder eine Gruppe -$COOR^{17}$, wobei $R^{17}$ genannte Bedeutung hat, darstellt, oder
$R^{14}$ Wasserstoff, Phenyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, Benzyl oder eine Gruppe der Formel

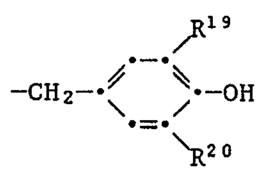

darstellt, wobei
$R^{19}$ und $R^{20}$, unabhängig voneinander -H, Methyl oder -$C(CH_3)_3$ bedeuten, mit der Massgabe, dass $R^{19}$ und $R^{20}$ nicht gleichzeitig -H sind, und
$R^5$ und $R^6$, unabhängig voneinander, Wasserstoff, $C_1$-$C_{12}$-Alkyl, durch mindestens ein -O- unterbrochenes $C_3$-$C_{20}$-Alkyl, $C_5$-$C_{12}$-Cycloalkyl, mit 1 bis 3 $C_{1-4}$-Alkylgruppen substituiertes Cycloalkyl mit 5 bis 12 Ring-C-Atomen, Phenyl, durch $C_1$-$C_{12}$-Alkyl, $C_1$-$C_4$-Alkoxy oder Halogen substituiertes Phenyl, $C_3$-$C_8$-Alkenyl, $C_7$-$C_{15}$-Aralkyl oder $C_2$-$C_4$-Hydroxyalkyl darstellen oder
$R^5$ und $R^6$ zusammen stellen Tetramethylen oder Pentamethylen oder durch -O-, -$NR^{21}$-oder -$S(O)_q$-unterbrochenes Tetramethylen oder Pentamethylen dar, wobei $R^{21}$ Wasserstoff, $C_1$-$C_{12}$-Alkyl oder Phenyl und q = 0, 1 oder 2 ist, oder, wenn n = 2 ist,
A eine Gruppe der Formel -O-X'-O- bedeutet, wobei
X' = $C_2$-$C_{10}$-Alkylen, $C_4$-$C_8$-Alkenylen, $C_4$-$C_8$-Alkinylen, Cyclohexylen, durch ein oder mehrere

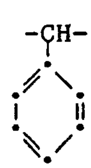 ,

-O-, -S(O)q oder -$NR^{22}$- unterbrochenes $C_4$-$C_{40}$-Alkylen, wobei $R^{22}$ Wasserstoff oder $C_1$-$C_{12}$-Alkyl oder Phenyl ist, und q = 0, 1 oder 2 bedeutet, oder
A eine Gruppe der Formel

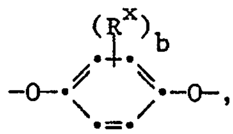

wobei $R^x$ eine Alkylgruppe mit 1 bis 8 C-Atomen darstellt und b = 0, 1 oder 2 ist, oder
A eine Gruppe der Formel

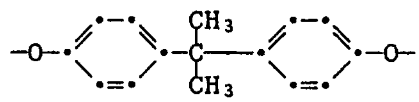

bedeutet, oder
A eine Gruppe der Formel

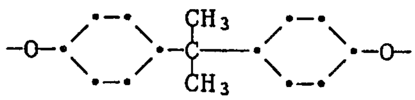

bedeutet, oder
A eine Gruppe der Formel
$-O-CH_2-CH(OH)-CH_2-O-X^2-O-CH_2-CH(OH)-CH_2-O-$ bedeutet,
wobei $X^2 = X'$ entspricht oder

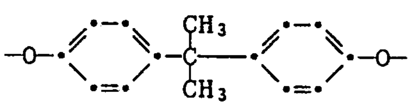

oder

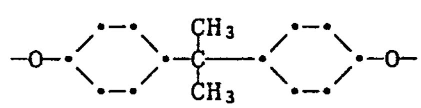

ist, oder
A eine Gruppe der Formel

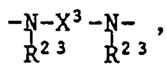

wobei
$X^3$ $C_2$-$C_{10}$-Alkylen und $R^{23}$ Wasserstoff, $C_1$-$C_{12}$-Alkyl oder Phenyl ist, bedeutet.

Die als zweckmässig und bevorzugt für A und n in Verbindungen der Formel I genannten Bedeutungen führen auch in neuen Verbindungen der Formel Ia zu zweckmässigen und bevorzugten Verbindungen.

Besonders bevorzugte neue Verbindungen der Formel Ia sind solche, worin
n =1 ist, und
A die Bedeutung von $-OR^4$ hat und
$R^4$ eine Alkylgruppe mit 1 bis 18 C-Atomen oder eine Alkenylgruppe mit 2 bis 18 C-Atomen darstellt, oder
n = 2 ist und
A die Bedeutung von $-O-X'-O-$ hat, wobei $X'$ = $C_2$-$C_8$-Alkylen oder $C_4$-$C_{12}$-Alkylen, das durch 1 bis 3 -O-, -S- oder $-NR^{22}-$ unterbrochen ist, oder Phenylen darstellt, wobei $R^{22}$ die Bedeutung von Wasserstoff, $C_1$-$C_{12}$-Alkyl oder Phenyl hat.

In anderen besonders bevorzugten neuen Verbindungen der Formel Ia ist
n = 1, und
$R^4$ hat die Bedeutung von $C_1$-$C_{18}$-Alkyl oder $C_{12}$-$C_{18}$-Alkenyl, oder
n ist = 2 und
A hat die Bedeutung von $-O-CH_2-CH_2-S-CH_2-CH_2-O-$ oder $-O-CH_2-CH_2-O-CH_2-CH_2-O-$.

Beispiele neuer Verbindungen sind demnach solche der Formel Ia, worin n gleich 1 ist und A die Bedeutung von $-OCH_3$, $-OC_8H_{17}$,

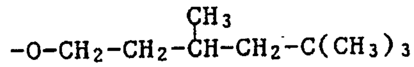

oder $-O-(CH_2)_8-CH=CH-(CH_2)_7-CH_3$ hat, oder worin n gleich 2 ist und A die Bedeutung von $-O-CH_2-CH_2-S-CH_2-CH_2-O-$oder $-O-CH_2-CH_2-O-CH_2-CH_2-$ hat.

Die Verbindungen der Formel I und der Formel Ia gemäss vorliegender Erfindung sind beispielsweise durch eine Michael-Addition erhältlich, wobei die Reaktion der nachstehenden Gleichung folgt:

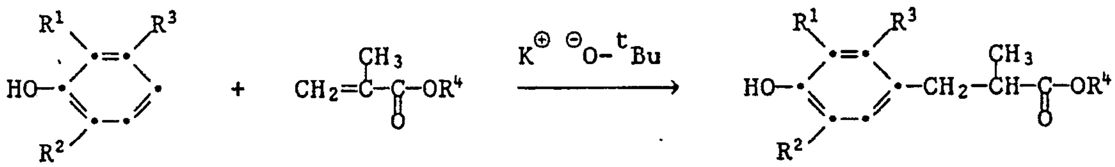


$R^1$, $R^2$, $R^3$ und $R^4$ können obengenannte Bedeutung haben.

Das Verfahren wird derart z.B. ausgeführt, dass das substituierte Phenol mit dem olefinischen Ester im stöchiometrischen Ueberschuss in Gegenwart einer katalytischen Menge einer Base, beispielsweise einer quaternären Ammoniumbase, eines Alkalimetallamides oder eines Alkalimetallalkoxides, bei einer Temperatur zwischen 150° und 220°C umgesetzt wird.

Eine detaillierte Verfahrensbeschreibung ist der eingangs erwähnten US-PS 4 529 809 zu entnehmen.

Auf diese Weise werden vor allem die Niederalkylester ($R^4$ = $C_1$-$C_4$-Alkyl, insbesondere Methyl), hergestellt. Die übrigen Ester können z.B. durch Umesterung nach der allgemeinen Gleichung erhalten werden:

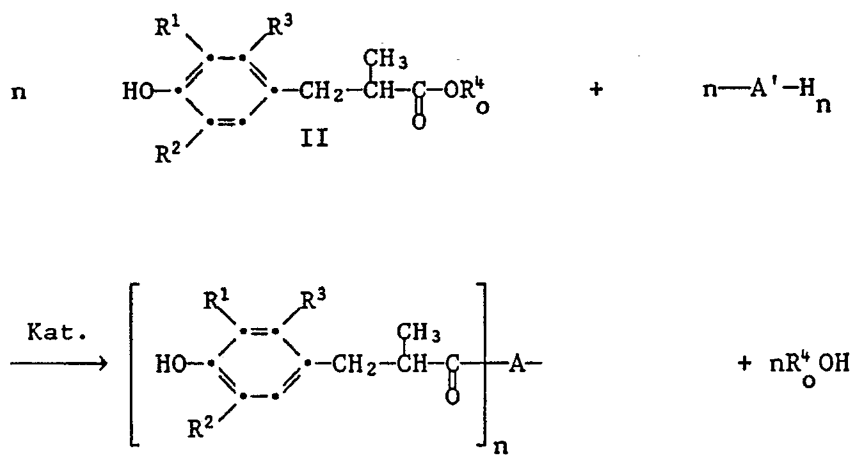


$R^1$, $R^2$ und $R^3$ haben obenstehende Bedeutung, während A′ für $-OR^4$ ohne $-C_1$-$C_4$-Alkyl oder-O-$X^1$-O- steht und $R^4_o$ $C_1$-$C_4$-Alkyl bedeutet.

Geeignete Katalysatoren sind z.B. Dibutylzinnoxid, $Ti(-O-i-C_3H_7)_4$, $LiNH_2$, LiH, LiOH, KOR, NaOR, LiOR, wobei R = H oder $C_1$-$C_{10}$-Alkyl ist, oder

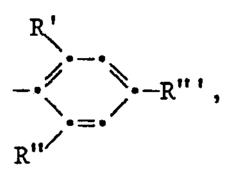

wobei R′, R″ und R‴, unabhängig voneinander, -H oder Alkyl mit 1 bis 10 C-Atomen sind.

Das Verfahren wird zweckmässig in einem Lösungsmittel, wie Benzol, Toluol, Xylol, Dimethylformamid usw. ausgeführt.

Ein weiteres Verfahren zur Herstellung der erfindungsgemässen Verbindungen folgt der allgemeinen Gleichung für die Veresterung:

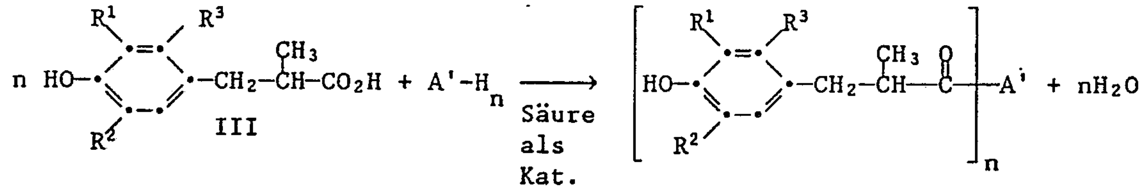


$R^1$, $R^2$, $R^3$ und A′ können dabei obengenannte Bedeutung haben. Die Reaktion wird in Gegenwart einer Säure als Katalysator durchgeführt. So kann als Katalysator beispielsweise eine Bleicherde, wie Tosil L80S, oder p-Toluolsulfonsäure zur Anwendung gelangen. Die Säuren der Formel III können z.B. durch Verseifung von Estern der Formel II erhalten werden.

Ein Verfahren, das geeignet ist, Verbindungen nach vorliegender Erfindung herzustellen, ist auch die Reaktion eines entsprechenden Säurechlorides mit einer Verbindung der Formel A-OH. Das Verfahren kann mit nachfolgender Reaktionsgleichung näher umschrieben werden:

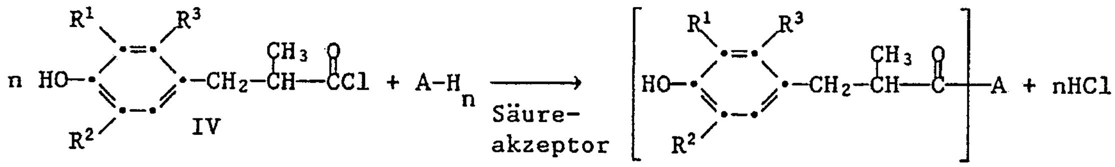


Die Bedeutung von $R^1$, $R^2$, $R^3$ und A ist oben angegeben. Als Säureakzeptor kann beispielsweise Pyridin, Triethylamin oder ein Alkalihydroxid, wie NaOH oder KOH eingesetzt werden. Die Verbindungen der Formel IV sind durch übliche Säurechlorierung von Verbindungen der Formel III erhältlich.

Ein anderes Verfahren zur Herstellung von Verbindungen der Formeln 1 und Ia nach vorliegender Erfindung folgt der Gleichung:

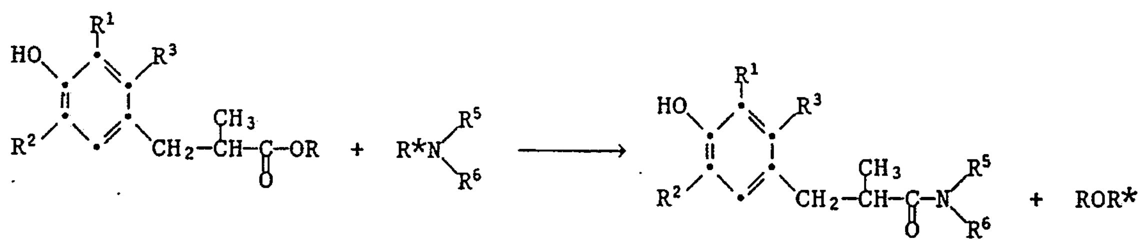

$R^1$, $R^2$, $R^3$, $R^5$ und $R^6$ sind in ihren Bedeutungen oben angegeben. R und R* haben, unabhängig voneinander, die Bedeutung von z.B. Wasserstoff oder $C_1$-$C_4$-Alkyl. Dabei erfolgt die Umsetzung durch thermische Reaktion bei beispielsweise 80-190 °C.

Die vorliegende Erfindung betrifft auch die Verwendung von Verbindungen der Formel I, wie oben beschrieben, als Stabilisatoren und/oder Kupplerlösungsmittel für farbphotographisches Aufzeichnungsmaterial.

Sofern Verbindungen der Formel (I) flüssig sind, können sie als Lösungsmittel für die photographischen Kuppler eingesetzt werden.

Ein weiterer Gegenstand vorliegender Erfindung ist die Verwendung von Verbindungen der Formel Ia, gemäss obiger Beschreibung, als Stabilisatoren und/oder Kupplerlösungsmittel für farbphotographisches Aufzeichnungsmaterial.

Gegebenenfalls können neben den als Stabilisatoren und/oder Kupplerlösungsmittel verwendeten Verbindungen der Formel I weitere an sich bekannte Stabilisatoren, beispielsweise Antioxidantien und Lichtschutzmittel und insbesondere phenolische Antioxidantien und sterisch gehinderte Amine, verwendet werden.

Hierdurch wird eine antioxidative Stabilisierung und eine Steigerung des Lichtschutzes bewirkt.

Beispiele für phenolische Antioxidantien sind weitere Verbindungen mit einer sterisch gehinderten Phenolgruppe. Die meisten dieser Verbindungen enthalten mindestens eine Gruppe der Formel

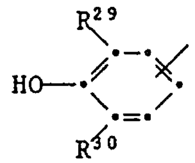

worin $R^{29}$ und $R^{30}$ unabhängig voneinander $C_1$-$C_{12}$-Alkyl, Cyclohexyl, Phenyl oder $C_7$-$C_9$-Phenylalkyl bedeuten und $R^{30}$ auch Wasserstoff bedeuten kann.

Bevorzugt sind Antioxidantien, die mindestens eine Gruppe der Formel

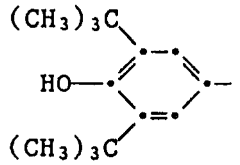

oder

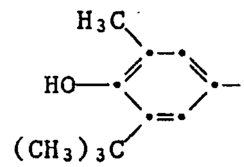

enthalten.

Beispiele für solche Antioxidantien sind die folgenden Verbindungen:

Alkylierte Monophenole , z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2,-(α-methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

Alkylierte Hydrochinone , z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

Hydroxylierte Thiodiphenylether , z.B. 2,2'-Thio-bis-(6-tert.butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

Alkyliden-Bisphenole , z.B 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat], Bis-(3-tert.butyl-4-hydroxy-5-methylphenyl)-dicyclopentadien, Bis-[2-(3'-tert.butyl-2'-hydroxy-5'-methylbenzyl)-6-tert.butyl-4-methyl-phenyl]-terephthalat.

Benzylverbindungen , z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-( 4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 1,3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

Ester der β-(3,5-Di-tert.butyl-4-hydroxyphenyl)-propionsäure mit ein-oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

Ester der β-(5-tert.Butyl-4-hydroxy3-methylphenyl)-propionsäure mit ein-oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

Ester der β-(3,5-Dicyclohexyl-4-hydroxyphenyl)-propionsäure mit ein-oder mehrwertigen Alkoholen, wie z.B. mit Methanol, Octadecanol, 1,6-Hexandiol, Heopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

Ester der 3,5-Di-tert.butyl-4-hydroxybenzoesäure mit ein- oder mehrwertigen Alkoholen oder Phenolen wie z.B. 2,4-Di-tert.butylphenol oder 2 ,4-Di-tert.pentylphenol.

Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trime-

thylendiamin, N,N′-Bis-(3,5-di-tert.butyl-4-hydroxyphenylpropionyl)-hydrazin.

Weitere Beispiele sind die Verbindungen der folgenden Formeln:

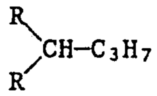
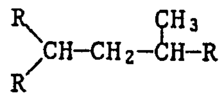
R = 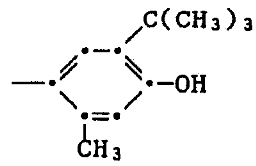

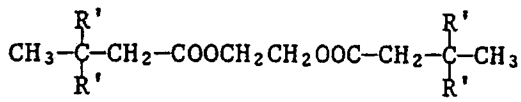
R' = 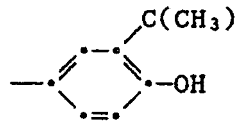

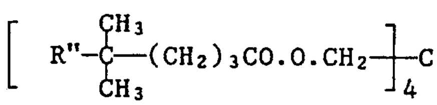
R" = 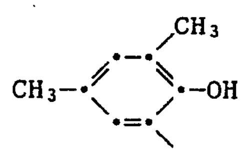

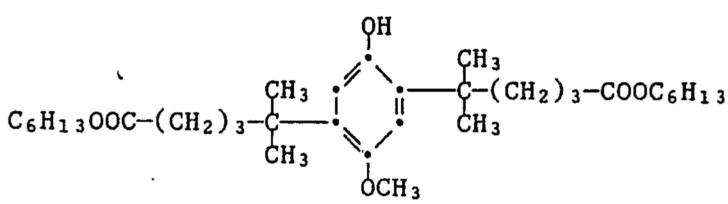

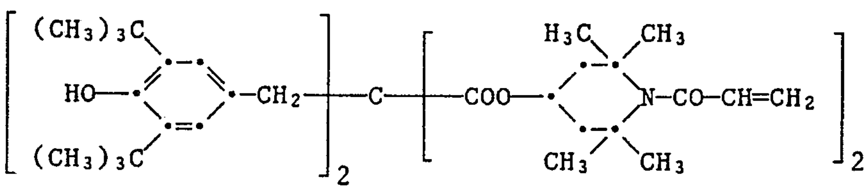

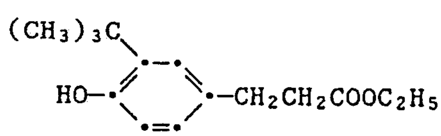
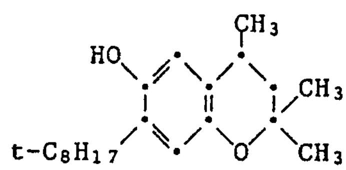

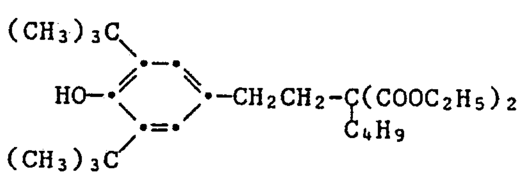

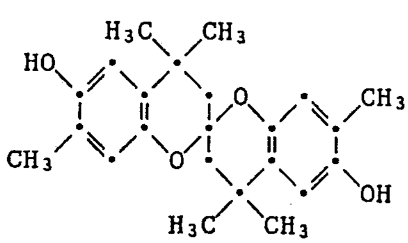
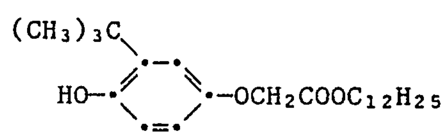

Anstelle der freien Phenole können auch verkappte Phenole verwendet werden Beispiele hierfür sind

veresterte, sulfonylierte, carbamoylierte oder silylierte Phenole. Während der Entwicklung werden daraus die freien Phenole gebildet.

Die photographischen Schichten können auch phenolische Verbindungen enthalten, die als Lichtschutzmittel für das Farbbild sowie als Mittel gegen Farbschleier wirken. Sie können in einer lichtempfindlichen Schicht (Farbschicht) oder in einer Zwischenschicht enthalten sein, allein oder zusammen mit anderen Additiven. Solche Verbindungen sind in den folgenden Patentschriften näher beschrieben: US-A-3,700,455, 3,591,381, 3,573,052, 4,030,931, 4,174,220, 4,178,184, 4,228,235, 4,279,990, 4,346,165, 4,366,226, 4,447,523, 4,528,264, 4,581,326, 4,562,146, 4,559,297, GB-A-1,309,277, 1,547,302, 2,023,862, 2,135,788, 2,139,370, 2,156,091; DE-A-2,301,060, 2,347,708, 2,526,468, 2,621,203, 3,323,448; DD-A-200,691, 214,468; EP-A-106,799, 113,124, 125,522, 159,912, 161,577, 164,030, 167,762, 176,845; JP-A-74/134,326, 76/127,730, 76/30462, 77/3822, 77/154,632, 78/10842, 79/48535, 79/70830, 79/73032, 79/147,038, 79/154,325, 79/155,836, 82/142,638, 83/224,353, 84/5246, 84/72443, 84/87456, 84/192,246, 84/192,247, 84/204,039, 84/204,040, 84/212,837, 84/220,733, 84/222,836, 84/228,249, 86/2540, 86,8843, 86/18835, 86/18836, 87/11456, 87/42245, 87/62157, 86/6652 sowie in Research Disclosure 79/17804.

Beispiele für sterisch gehinderte Amine als Lichtschutzmittel sind die einsetzbaren Polyalkylpiperidin-Lichtschutzmittel, die niedermolekular (MG < 700) oder höhermolekular (Oligomere, Polymere) sein können. Sie besitzen die charakteristische Gruppe A

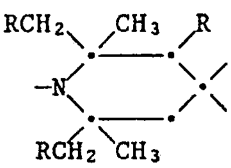

(A)

worin R Wasserstoff oder Methyl bedeutet. Diese Gruppe kann im Molekül einmal oder mehrmals vorhanden sein. Bevorzugt sind Piperidinderivate mit der Gruppe A, worin R Wasserstoff ist. Es handelt sich dabei um Derivate des 2,2,6,6-Tetramethylpiperidins. Bevorzugt tragen diese Polyalkylpiperidine einen oder zwei polare Substituenten in 4-Stellung oder ein polares Spiro-Ringsystem ist an die 4-Stellung gebunden.

Eine ausführliche Beschreibung derartiger Lichtschutzmittel und deren bevorzugter Formen sind z.B. aus der EP-A-0 290 391 verfügbar.

Das Gewichtsverhältnis von Verbindungen der Formel I zu weiteren Antioxidantien und Lichtschutzmitteln kann in einem breiten Bereich variieren. Bevorzugt beträgt das Gewichts-Verhältnis von Verbindungen der Formel I zu den weiteren Antioxidantien und Lichtschutzmitteln 1:10 bis 10:1, insbesondere 1:5 bis 3:1.

Sofern die Verbindungen der Formel I und gegebenenfalls weitere Stabilisatoren wasserlöslich sind, kann man sie als wässrige Lösung der photographischen Gelatineschicht bei deren Zubereitung zusetzen. Viele dieser Verbindungen sind jedoch in Wasser nur sehr wenig löslich und man löst sie dann in einem organischen Lösungsmittel bzw. Lösungsmittelgemisch und emulgiert die Lösung in einer Gelatinelösung 7 die dann zur photographischen Gelatineschicht bei deren Zubereitung zugesetzt wird. Als Lösungsmittel verwendet man bevorzugt ein Gemisch eines niedrig-siedenden und eines hoch-siedenden Lösungsmittels und entfernt das niedrig-siedende Lösungsmittel während der Emulgierung. Beispiele für verwendbare niedrig-siedende Lösungsmittel sind Methylacetat, Ethylacetat, Tetrachlormethan, Dichlormethan, Trichlormethan, Methanol, Ethanol, Dioxan, Aceton oder Benzol. Beispiele für hoch-siedende Lösungsmittel sind Dimethylformamid, Dimethylsulfoxid, Dialkylphthalate oder Triarylphosphate.

Das Dispergieren der Stabilisatorlösung in der Gelatinelösung kann z.B. in einer Kolloidmühle oder in einem Homogenisator oder mit Hilfe von Ultraschall geschehen. Hierbei können auch oberflächenaktive Mittel (Emulgatoren) zugesetzt werden. Eine feine Dispergierung ist Voraussetzung für die homcgene Verteilung der Stabilisatoren in der photographischen Schicht.

Die Zusatzmenge an Verbindung der Formel I beträgt pro Schicht im allgemeinen bis zu 1 g/m$^2$, vorzugsweise 10-300 mg/m$^2$. Falls eine Kombination von Verbindungen der Formel I mit weiteren Stabilisatoren verwendet wird, so beträgt die Zusatzmenge insgesamt zweckmässig ebenfalls bis zu 1 g/m$^2$ und vorzugsweise 10-300 mg/m$^2$.

Der Zusatz kann z.B. zu einer oder zwei oder allen drei Farbsilberschichten erfolgen. Von besonderer Bedeutung ist der Zusatz zur Gelbschicht. In den Schichten befindet sich das sensibilisierte Silberhalogenid und der jeweilige Farbkuppler. Ausserdem können die Schichten weitere Stabilisatoren und/oder sonstige Zusätze enthalten.

Die Gelbkuppler sind vorzugsweise Verbindungen der Formel IX,

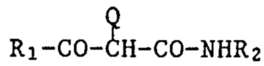 IX

worin $R_1$ Alkyl oder Aryl ist, $R_2$ Aryl ist und Q Wasserstoff oder eine Gruppe ist, die durch Reaktion mit dem oxidierten Entwickler abgespalten werden kann.

Eine Gruppe von Gelbkupplern sind solche Verbindungen der Formel IX, in denen $R_1$ tert-Butyl ist und $R_2$ eine Gruppe der Formel

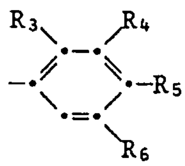

ist, worin $R_3$ Wasserstoff, Halogen, Alkyl oder Alkoxy bedeutet und $R_4$, $R_5$ und $R^6$ Wasserstoff, Halogen, Alkyl, Alkenyl, Alkoxy, Aryl, Carboxy, Alkoxycarbonyl, eine Carbamylgruppe, eine Sulfon- oder Sulfamylgruppe, eine Alkylsulfonamidogruppe, Acylaminogruppe, Ureidogruppe oder Aminogruppe bedeuten.

Vorzugsweise sind $R_3$ Chlor, $R_4$ und $R_5$ Wasserstoff und $R_6$ eine Acylaminogruppe. Hierzu gehören auch die Verbindungen der Formel

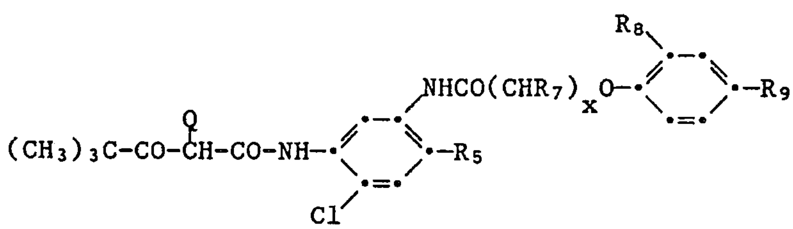

worin x 1-4 ist, $R_5$ Wasserstoff oder Alkyl ist und $R_8$ und $R_9$ Alkyl sind.

Eine andere Gruppe von Gelbkupplern entspricht der Formel X

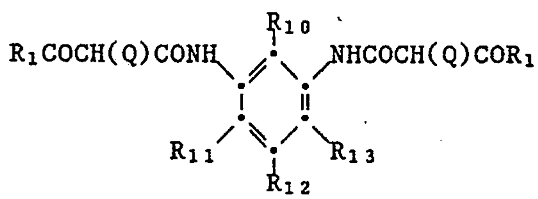 X

worin $R_{10}$ Wasserstoff, Halogen oder Alkoxy ist,
$R_{11}$, $R_{12}$ und $R_{13}$ Wasserstoff, Halogen, Alkyl, Alkenyl, Alkoxy, Aryl, Carboxyl, Alkoxycarbonyl, eine Carbamylgruppe, eine Sulfongruppe, Sulfamylgruppe, Sulfonamidogruppe, Acylaminogruppe, Ureidogruppe oder Aminogruppe bedeuten und $R_1$ und Q die oben angegebene Bedeutung haben.

Dazu gehören Verbindungen der Formel X, in denen $R_1$ tert.Butyl ist, $R_{10}$ Chlor ist, $R_{11}$ und $R_{13}$ Wasserstoff sind und $R_{12}$ Alkoxycarbonyl ist.

In den Verbindungen der Formel IX und X kann die Abgangsgruppe Q Wasserstoff oder Halogen sein oder sie ist eine heterocyclische Gruppe

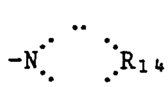

worin $R_{14}$ eine organische zweiwertige Gruppe ist, die den Ring zu einem 4-7-gliedrigen Ring ergänzt oder Q ist eine Gruppe $-OR_{15}$, worin $R_{15}$ Alkyl, Aryl, Acyl oder ein heterocyclischer Rest ist.

Typische Beispiele für gebräuchliche Gelbkuppler sind die Verbindungen der folgenden Formeln:

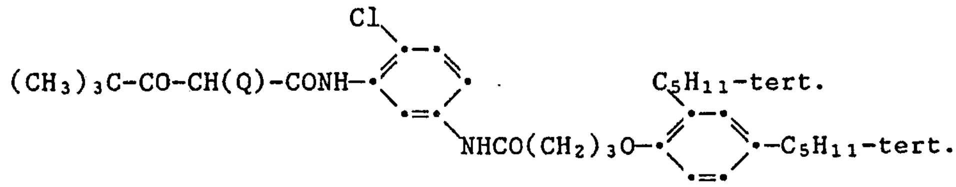

a) Q = 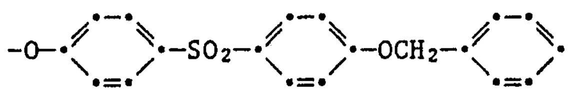

b) Q = 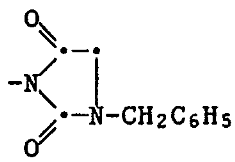

c) Q = 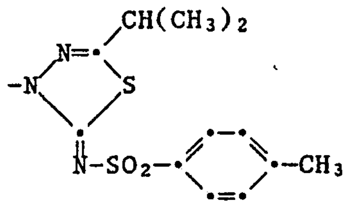

d) Q = 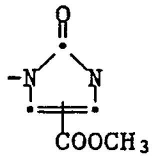

e) Q = 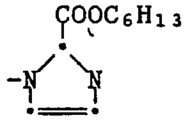

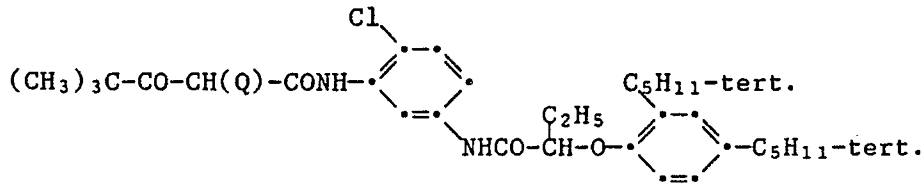

f) Q = 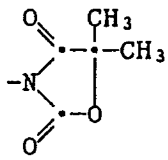

g) Q = 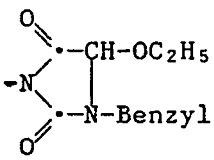

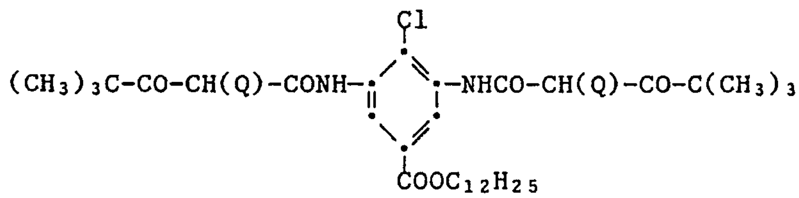

h) Q = 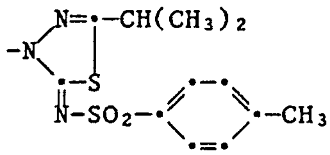

Weitere Beispiele für Gelbkuppler sind zu finden in den US-A 2,407,210, 2,778,658, 2,875,057, 2,908,513, 2,908,573, 3,227,155, 3,227,550, 2,253,924, 3,265,506, 3,277,155, 3,408,194, 3,341,331, 3,369,895, 3,384,657, 3,415,652, 3,447,928, 3,551,155, 3,582,322, 3,725,072, 3,891,445, 3,933,501 ,4,115,121, 4,401,752, 4,022,620, in den DE-A 1,547,868, 2,057,941, 2,162,899, 2,163,813, 2,213,461, 2,219,917, 2,261,361, 2,261,362, 2,263,875, 2,329,587, 2,414,006, 2,422,812 und in den GB-A 1,425,020 und 1,077,874.

Die Gelbkuppler werden üblicherweise in einer Menge von 0,05-2 Mol und vorzugsweise 0,1-1 Mol pro Mol Silberhalogenid verwendet.

Magenta-Kuppler können z.B. einfache 1-Aryl-5-pyrazolone sein oder mit 5-gliederigen Heteroringen kondensierte Pyrazolderivate wie z.B Imidazopyrazole, Pyrazolopyrazole, Pyrazolotriazole oder Pyrazolotetrazole.

Eine Gruppe von Magentakupplern sind 5-Pyrazolone der Formel XI,

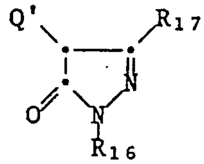

XI

wie sie in der Britischen Patentschrift 2,003,473 beschrieben sind. Darin ist $R_{16}$ Wasserstoff, Alkyl, Aryl, Alkenyl oder eine heterocyclische Gruppe. $R_{17}$ ist Wasserstoff, Alkyl, Aryl, eine heterocyclische Gruppe, eine Estergruppe, Alkoxygruppe, Alkylthiogruppe Carboxylgruppe, Arylaminogruppe, Acylaminogruppe,

(Thio)-harnstoffgruppe, (Thio)-carbamoylgruppe, Guanidinogruppe oder Sulfonamidogruppe.

Bevorzugt ist $R_{17}$ eine Gruppe

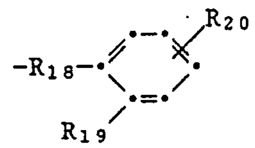

worin $R_{18}$ Imino, Acylamino oder Ureido ist, $R_{19}$ Wasserstoff, Halogen, Alkyl oder Alkoxy ist, $R_{20}$ Wasserstoff, Alkyl, Acylamino, Carbamoyl, Sulfamoyl, Sulfonamido, Alkoxycarbonyl, Acyloxy oder eine Urethangruppe ist.

Wenn $Q'$ Wasserstoff ist, so ist der Magentakuppler tetraäquivalent in bezug auf das Silberhalogenid.

Typische Beispiele für diesen Typ von Magentakupplern sind Verbindungen der Formel

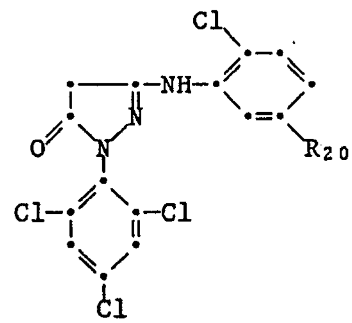

worin $R_{20}$ die oben genannten Bedeutungen hat.

Weitere Beispiele solcher tetraäquivalenter Magentakuppler sind zu finden in den US-A 2,983,608, 3,061,432, 3,062,653, 3,127,269, 3,152,896, 3,311,476, 3,419,391, 3,519,429, 3,558,319, 3,582,322, 3,615,506, 3,684,514, 3,834,908, 3,888,680, 3,891,445, 3,907,571, 3,928,044, 3,930,861, 3,930,866, 3,933,500.

Wenn $Q'$ in Formel XI nicht Wasserstoff ist sondern eine Gruppe, die bei der Reaktion mit dem oxidierten Entwickler eliminert wird, so handelt es sich um einen diäquivalenten Magentakuppler. $Q'$ kann in diesem Fall z.B. Halogen oder eine über O, S oder N an den Pyrazolring gebundene Gruppe sein. Solche diäquivalente Kuppler ergeben eine höhere Farbdichte und sind reaktiver gegenüber dem oxidierten Entwickler als die entsprechenden tetraäquivalenten Magentakuppler. Bevorzugt ist $Q'$ eine O-Alkoxyarylthio-Gruppe.

Beispiele für diäquivalente Magentakuppler sind beschrieben in den US-A 3,006,579, 3,419,391, 3,311,476, 3,432,521, 3,214,437, 4,032,346, 3,701,783, 4,351,897, 3,227,554, im EP-A-133,503, DE-A-2,944,601, JP-A-78/34044, 74/53435, 74/53436, 75/53372 und 75/122935.

Ueber ein zweiwertiges $Q'$ können 2 Pyrazolonringe verknüpft werden und man erhält dann sogenannte Bis-Kuppler. Solche sind z.B. beschrieben im US-A-2,632,702, US-A-2,618,864, GB-A-968,461, GB-A-786,859, JP-A-76/37646, 59/4086, 69/16110, 69/26589, 74/37854 und 74/29638.

Wie vorhin erwähnt können als Magentakuppler auch mit 5-gliedrigen Heterocyclen kondensierte Pyrazole - sogenannte Pyrazoloazole - verwendet werden. Deren Vorteile gegenüber einfachen Pyrazolen ist, dass sie Farben von grösserer Formalin-Beständigkeit und reineren Absorptionsspektren aufweisen.

Man kann sie durch die allgemeine Formel XII darstellen,

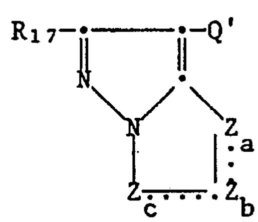

XII

worin $Z_a$, $Z_b$ und $Z_c$ die Ergänzungen zu einem 5-gliedrigen Ring bedeuten, der bis zu 4 Stickstoffatome enthalten kann. Die Verbindungen können demgemäss Pyrazolo-imidazole, Pyrazolo-pyrazole, Pyrazolo-triazole oder Pyrazolo-tetrazole sein. $R_{17}$ und $Q'$ haben dieselben Bedeutungen wie in Formel XI.

Pyrazolo-tetrazole sind beschrieben in der JP-A-85/33552; Pyrazolo-pyrazole in der JP-A-85/43,695; Pyrazolo-imidazole in den JP-A-85/35732, JP-A-86/18949 und US-A-4,500,630; Pyrazolo-triazole in den JP-A-85/186,567, JP-A-86/47957, JP-A-85/215,687, JP-A-85/197,688, JP-A-85/172,982, EP-A-119,860, EP-A-173,256, EP-A-178,789, EP-A-178,788 und in Research Disclosure 84/24,624.

Weitere Pyrazoloazol-Magentakuppler sind beschrieben in: JP-A-86/28,947, JP-A-85/140,241, JP-A-85/262,160, JP-A-85/213,937, EP-A-177,765, EP-A-176,804, EP-A-170,164, EP-A-164,130, EP-A-178,794, DE-A-3,516,996, DE-A-3,508,766 und Research Disclosure 81/20919, 84/24531 und 85/25758.

Cyankuppler können z.B. Derivate von Phenol, von 1-Haphthol oder von Pyrazolochinazolon sein. Bevorzugt sind Strukturen der Formel XIII,

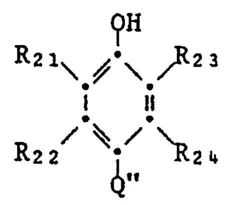

XIII

worin $R_{21}$, $R_{22}$, $R_{23}$ und $R_{24}$ Wasserstoff, Halogen,Alkyl, Carbamoyl, Amido, Suflonamido, Phosphoramido oder Ureido sind. $R^{21}$ ist vorzugsweise H oder Cl, $R_{22}$ ist vorzugsweise eine Alkyl- oder Amidogruppe. $R_{23}$ ist vorzugsweise eine Amidogruppe und $R_{24}$ ist vorzugsweise Wasserstoff. $Q''$ ist Wasserstoff oder eine Abgangsgruppe, die bei der Reaktion mit dem oxidierten Entwickler abgespalten wird. Eine ausführliche Aufzählung von Cyankupplern ist in der US-A-4,456,681 zu finden.

Beispiele von gebräuchlichen Cyankupplern sind die folgenden:

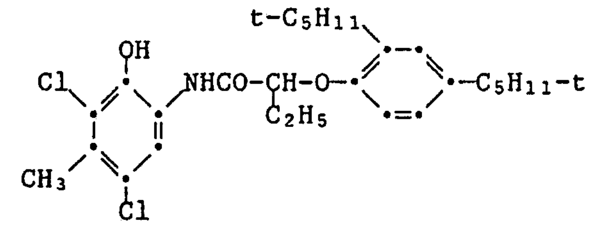

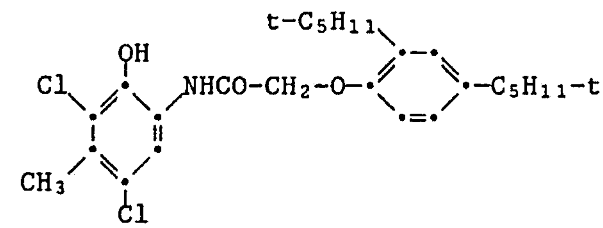

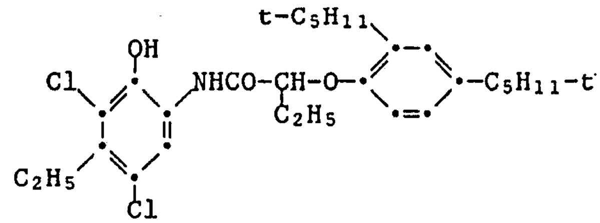

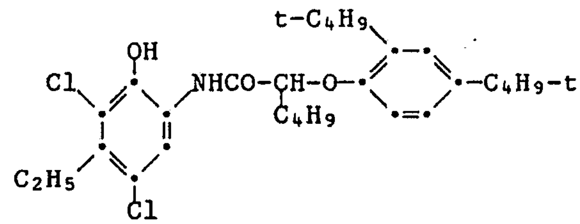

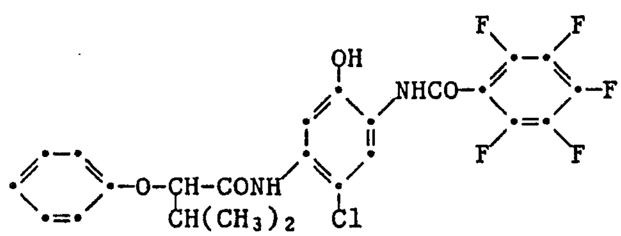

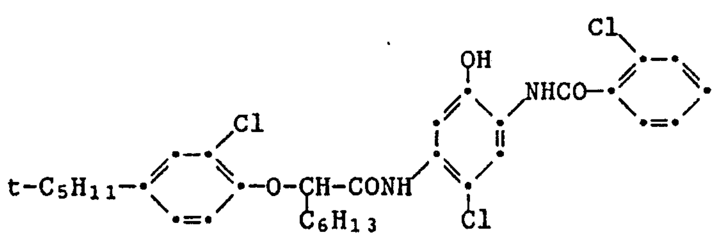

Weitere Beispiele von Cyankupplern sind in folgenden US-Patentschriften zu finden: 2,369,929, 2,423,730, 2,434,272, 2,474,293, 2,521,908, 2,698,794, 2,706,684, 2,772,162, 2,801,171, 2,895,826, 2,908,573, 3,034,892, 3,046,129, 3,227,550, 3,253,294, 3,311,476, 3,386,301, 3,419,390, 3,458,315, 3,476,560, 3,476,563, 3,516,831, 3,560,212, 3,582,322, 3,583,971, 3,591,383, 3,619,196, 3,632,347, 3,652,286, 3,737,326, 3,758,308, 3,839,044, 3,880,661, 4,004,929, 4,124,396, 4,333,999, 4,463,086, 4,456,681.

Die für farbfotographische Materialien üblicherweise verwendeten Farbentwickler sind p-Dialkylaminoaniline. Beispiele hierfür sind 4-Amino-N,N-diethylanilin, 3-Methyl-4-amino-N,N-diethylanilin, 4-Amino-N-ethyl-N-α-hydroxyethylanilin, 3-Methyl-4-amino-N-ethyl-N-α-hydroxyethylanilin, 3-Methyl-4-amino-N-ethyl-N-α-methanesulphonamidoethylanilin 3-Methyl-4-amino-N-ethyl-N-α-methoxyethyl-anilin, 3-α-Methansulphonamidoethyl-4-amino-N,N-diethylanilin, 3-Methoxy-4amino-N-ethyl-N-α-hydroxyethyl-anilin, 3-

Methoxy-4-amino-N-ethyl-N-α-methoxyethylanilin, 3-Acetamido-4-amino-N,N-diethylanilin, 4-Amino-N,N-dimethylanilin, N-Ethyl-N-α-(α-(α-methoxyethoxy)ethoxy]ethyl-3-methyl-4-aminoanilin, N-Ethyl-N-α-(α-methoxyethoxy)ethyl-3-methyl-4-aminoanilin sowie die Salze solcher Verbindungen, wie z.B. Sulfate, Hydrochloride oder Toluolsulfonate.

Die erfindungsgemäss verwendbaren Verbindungen der Formel I können zusammen mit dem Farbkuppler und gegebenenfalls weiteren Zusätzen in das farbphotographische Material eingearbeitet werden, indem man sie in hochsiedenden organischen Lösungsmitteln vorlöst. Vorzugsweise verwendet man Lösungsmittel, die höher als 160 °C sieden. Typische Beispiele solcher Lösungsmittel sind die Ester von Phthalsäure, Phosphorsäure, Zitronensäure, Benzoesäure oder von Fettsäuren, sowie Alkylamide und Phenole.

Meist verwendet man zusätzlich noch ein niedrig siedendes Lösungsmittel, um das Einarbeiten der Zusätze in das farbphotographische Material zu erleichtern. Beispiele für solche Lösungsmittel sind Ester wie z.B. Ethylacetat, Alkohole wie z.B. Butanol, Ketone wie z.B. Methyl-isobutylketon, Chlorkohlenwasserstoffe wie z.B. Methylenchlorid, oder Amide wie z.B. Dimethylformamid. Sind die Zusätze selbst flüssig, so kann man sie auch ohne Zuhilfenahme von Lösungsmitteln in das Photomaterial einarbeiten.

Weitere Details über verwendbare hochsiedende Lösungsmittel sind in den folgenden Patentschriften zu finden.

Phosphate: GB-A-791,219, BE-A-755,248, JP-A-76/76739, 78/27449, 78/218,252, 78/97573, 79/148,113, 82/216,177, 82/93323 und 83/216,177.

Phthalate: GB-A-791,219, JP-A-77/98050, 82/93322, 82/216,176, 82/218,251, 83/24321, 83/45699, 84/79888.

Amide: GB-A-791,219, JP-A-76/105,043, 77/13600, 77/61089, 84/189,556, US-A-928,741.

Phenole: GB-A-820,329, FR-A-1,200,657, JP A-69/69946, 70/3818, 75/123,026, 75/82078, 78/17914, 78/21166, 82/212,114 und 83/45699.

Andere sauerstoffhaltige Verbindungen: US-A-3,748,141, 3,779,765, JP-A-73/75126, 74/101,114, 74/10115, 75/101,625, 76/76740, 77/61089 und BE-A-826,039.

Sonstige Verbindungen: JP-A-72/115,369, 72/130,258, 73/127,521, 73/76592, 77/13193, 77/36294, 79/95233 und Research Disclosure 82/21918.

Die Menge von hochsiedendem Lösungsmittel liegt zweckmässig im Bereich von 0,1 bis 300 %, vorzugsweise 10 bis 100 %, bezogen auf den Farbkuppler.

Die photographischen Schichten können ferner Farbschleier-Inhibitoren enthalten. Diese verhindern das Entstehen von Farbschleiern, wie sie beispielsweise durch Reaktion des Kupplers mit unabsichtlich oxidiertem Entwickler oder mit Nebenprodukten des Farbbildungsprozesses entstehen. Solche Farbschleierinhibitoren sind meist Hydrochinonderivate, können aber auch Derivate von Aminophenolen, von Gallussäure oder von Ascorbinsäure sein. Typische Beispiele hierfür sind in folgenden Patentschriften zu finden: US-A-2,360,290, 2,336,327, 2,403,721, 2,418,613, 2,675,314, 2,701,197, 2,704,713, 2,728,659, 2,732,300, 2,735,365; EP-A-124,877; JP-A-75/92988, 75/92989, 75/93928, 75/110,337 und 77/146,235.

Die photographischen Schichten können auch sogenannte DIR-Kuppler enthalten, die mit dem oxidierten Entwickler farblose Verbindungen ergeben. Sie werden zugesetzt zur Verbesserung der Schärfe und Körnigkeit der Farbbilder.

Die photographischen Schichten können auch UV-Absorber enthalten. Diese filtern das UV-Licht aus und schützen damit die Farbstoffe, die Kuppler oder sonstige Komponenten gegen Lichtabbau. Beispiele für solche UV-Absorber sind 2-(2-Hydroxyphenyl)-benztriazole, 2-Hydroxybenzophenone, Salicylsäureester, Acrylnitrilderivate oder Thiazoline. Solche UV-Absorber sind z.B. in folgenden Patentschriften näher aufgeführt:

US-A-3,314,794, 3,352,681, 3,705,805, 3,707,375, 4,045,229, 3,700,455, 3,533,794, 3,698,907, 3,705,805, 3,738,837 und JP-A-71/2784. Bevorzugte UV-Absorber sind die 2-(2-Hydroxyphenol)-benztriazole.

Die photographischen Schichten können auch gewisse Phosphor-III-Verbindungen, insbesondere Phosphite und Phosphonite, enthalten. Diese fungieren als Lichtschutzmittel für die Farbbilder sowie als Dunkellager-Stabilisator für Magentakuppler. Man setzt sie vorzugsweise den hochsiedenden Lösungsmitteln zu, zusammen mit dem Kuppler. Solche Phosphor-III-Verbindungen sind in den folgenden Patentschriften näher beschrieben: US-A-4,407,935, US-A-4,436,811, EP-A-181,289, JP-A-73/32728, JP-A-76/1420 und JP-A-55/67741.

Die photographischen Schichten können auch metallorganische Komplexe enthalten, die Lichtschutzmittel für die Farbbilder sind, insbesondere für die Magenta-Farbstoffe. Solche Verbindungen und deren Kombination mit anderen Additiven sind in folgenden Patentschriften näher beschrieben:

US-A-4,050,938, 4,239,843, 4,241,154, 4,242,429, 4,241,155, 4,242,430, 4,273,854, 4,246,329, 4,271,253, 4,242,431, 4,248,949, 4,245,195, 4,268,605, 4,246,330, 4,269,926, 4,245,018, 4,301,223, 4,343,886, 4,346,165, 4,590,153; JP-A-81/167,138, 81/168,652, 82/30834, 82/161,744; EP-A-137,271, 161,577, 185,506;

DE-A-2,853,865.

Die photographischen Schichten können auch Hydrochinonverbindungen enthalten. Diese wirken als Lichtschutzmittel für die Farbkuppler und für die Farbbilder sowie als Abfänger von oxidiertem Entwickler in Zwischenschichten. Sie werden vor allem in der Magentaschicht verwendet. Solche Hydrochinon-Verbindungen und deren Kombinationen mit anderen Additiven sind in folgenden Patentschriften näher beschrieben: US-A-2,360,290, 2,336,327, 2,403,721, 2,418,613, 2,675,314, 2,701,197, 2,710,801, 2,732,300, 2,728,659, 2,735,765, 2,704,713, 2,937,086, 2,816,028, 3,582,333, 3,637,393, 3,700,453, 3,960,570, 3,935,016, 3,930,866, 4,065,435, 3,982,944, 4,232,114, 4,121,939, 4,175,968, 4,179,293, 3,591,381, 3,573,052, 4,279,990, 4,429,031, 4,346,165, 4,360,589, 4,346,167, 4,385,111, 4,416,978, 4,430,425, 4,277,558, 4,489,155, 4,504,572, 4,559,297, FR-A-885,982; GB-A-891,158, 1,156,167, 1,363,921, 2,022,274, 2,066,975, 2,071,348, 2,081,463, 2,117,526, 2,156,091; DE-A-2,408,168, 2,726,283, 2,639,930, 2,901,520, 3,308,766, 3,320,483, 3,323,699; DD-A-216,476, 214,468, 214,469, EP-A-84290, 110,214, 115,305, 124,915, 124,877, 144,288, 147,747, 178,165, 161,577; JP-A-75/33733, 75/21249, 77/128,130, 77/146,234, 79/70036, 79/133,131. 81/83742, 81/87040, 81/109,345, 83/134,628, 82/22237, 82/112,749, 83/17431, 83/21249, 84/75249, 84/149,348, 84/182,785, 84/180,557, 84/189,342, 84/228,249, 84/101,650, 79/24019, 79/25823, 86/48856, 86/48857, 86/27539, 86/6652, 86/72040, 87/11455, 87/62157, sowie in Research Disclosure 79/17901, 79/17905, 79/18813, 83/22827 und 84/24014.

Die photographischen Schichten können auch Derivate von Hydrochinonethern enthalten. Diese Verbindungen wirken als Lichtschutzmittel und sind besonders geeignet zur Stabilisierung von Magenta-Farbstoffen. Solche Verbindungen und deren Kombination mit anderen Additiven sind in folgenden Patentschriften näher beschrieben:
US-A 3,285,937, 3,432,300, 3,519,429, 3,476,772, 3,591,381, 3,573,052, 3,574,627, 3,573,050, 3,698,909, 3,764,337, 3,930,866, 4,113,488, 4,015,990, 4,113,495, 4,120,723, 4,155,765, 4,159,910, 4,178,184, 4,138,259, 4,174,220, 4,148,656, 4,207,111, 4,254,216, 4,314,011, 4,273,864, 4,264,720, 4,279,990, 4,332,886, 4,436,165, 4,360,589, 4,416,978, 4,385,111, 4,459,015, 4,559,297; GB-A 1,347,556, 1,366,441, 1,547,392, 1,557,237, 2,135,788; DE-A 3,214,567; DD-214,469, EP-A 161,577, 167,762, 164,130, 176,845; JP-A 76/123,642, 77/35633, 77/147,433, 78/126, 78/10430, 78/53321, 79/24019, 79/25823, 79/48537, 79/44521, 79/56833, 79/70036, 79/70830, 79/73032, 79/95233, 79/145,530, 80/21004, 80/50244, 80/52057, 80/70840, 80/139,383, 81/30125, 81/151,936, 82/34552, 82/68833, 82/204,036, 82/204,037, 83/134,634, 83/207,039, 84/60434, 84/101,650, 84/87450, 84/149,348, 84/182,785, 86/72040, 87/11455, 87/62157, 87/63149, 86/2151, 86/6652, 86/48855 sowie in Research Disclosure 78/17051.

Die photographischen Schichten können auch phenolische Thianderivate gemäss der EP-A-0 310 551 und/oder Tetrahydrothiopyranverbindungen gemäss der EP-A 0 310 552 als Stabilisatoren enthalten.

Die folgenden Beispiele erläutern die Erfindung näher. Teile und Prozente bedeuten darin Gewichtsteile und Gewichtsprozente. Die Temperaturen sind in °C angegeben.

Beispiele:

1. Herstellung von 3-[3,5-di-tert.Butyl-4-hydroxyphenyl ]-2-methylpropansäuremethylester

Analog dem Stande der Technik, beispielsweise gemäss Beispiel 1 aus der US 4 529 089 aus 2,6-Di-tert.-butylphenol und Methylmethacrylat.

2. 3-(3',5'-di-tert-butyl-4'-hydroxyphenyl)-2-methylpropansäureoctadecylester

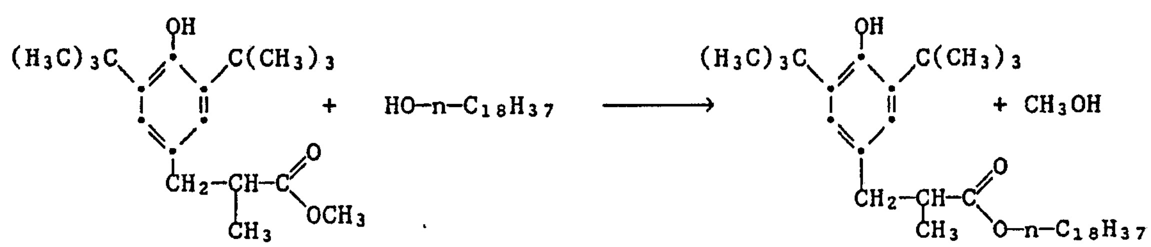

30,6 g (0,1 Mol) Methyl-2-methyl-3-(3',5'-di-tert-butyl-4'-hydroxyphenyl)- propionat und 27 g (0,1 Mol) 1-n-Octadecanol werden in einem 4-Hals-Glaskolben vorgelegt und auf 100 ° C aufgeheizt. Dann gibt man 0,25 g (1 Mol-%) Dibutylzinnoxyd zu und erhitzt bis auf 180 ° C. Dabei spaltet sich Methanol ab. Die Temperatur wird während 6 Stunden auf 180 ° C gehalten.

Das Reaktionsgemisch wird über Kieselgel chromatographiert.

Man erhält 52,4 g ≙ 96,2 % d.Th. eines Oeles, das langsam kristallisiert.

| | | |
|---|---|---|
| Analyse: berechnet | 79,35 % C | 11,84 % H |
| gefunden | 79,57 % C | 11,68 % H |

3. Man geht wie in Beispiel 2 vor, verwendet aber anstatt 1-n-Octadecanol die Verbindung der Formel

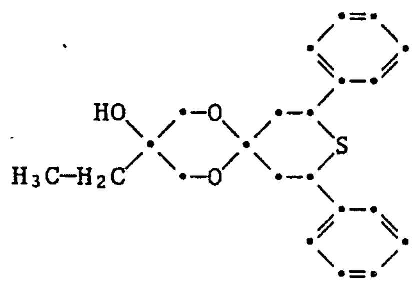

Man erhält so die Verbindung der Formel

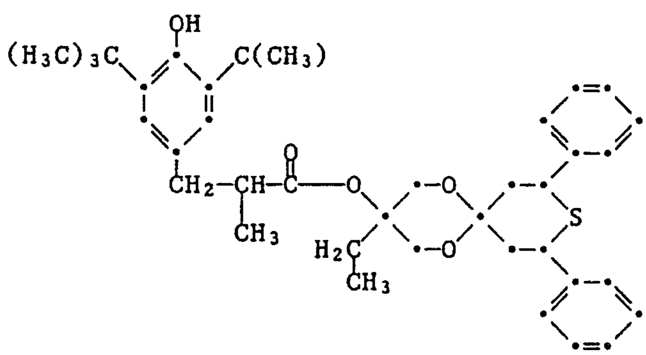

als gelbes Oel.

4. 3-(3',5'-di-t-butyl-4'-hydroxyphenyl)-2-methylpropansäure-2-ethylhexylester

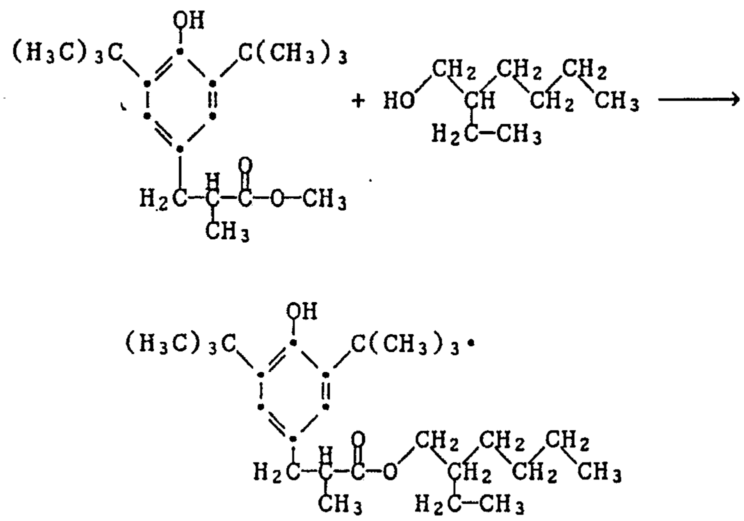

61,3 g (0,2 Mol) Methyl-2-methyl-3-(3′,5′-di-t-butyl-4′-hydroxyphenyl) propionat und 26 g (0,2 Mol) 2-Ethyl-1-hexanol werden in einem 4-Hals-Glaskolben vorgelegt und auf 100°C aufgeheizt. Dann wird 1 g (2 Mol-%) Dibutylzinnoxyd zugegeben und die Temperatur auf 180°C gesteigert. Es spaltet sich Methanol ab. Das Reaktionsgemisch wird während 9 Stunden bei 180°C gehalten. Das Produkt wird in einer Ausbeute von 79,4 g ≙ 98,2 % d.Th., als Harz erhalten. Zur Reinigung wird das Produkt über Kieselgel chromatographiert.

| | | | |
|---|---|---|---|
| Analyse: berechnet | 77,18 % C | 10,96 % H | 11,86% |
| gefunden | 77,05 % C | 11,03 % H | - |

5. Thiodiethylenglykol-bis[2-methyl-3-(3′,5′-di-t-butyl-4′-hydroxyphenyl)-propionat]

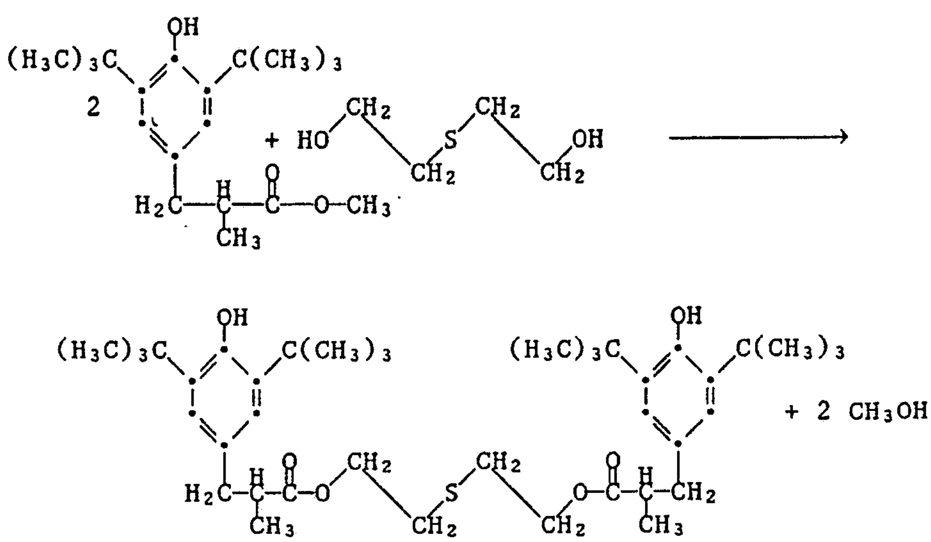

91,9 g (0,3 Mol) Methyl-2-methyl-3-(3′,5′-di-t-butyl-4′-hydroxyphenyl) propionat und 18,3 g (0,15 Mol) Thiodiethylenglykol werden in einem 4-Hals-Glaskolben vorgelegt und auf 100°C aufgeheizt. Man gibt 0,4 g Dibutylzinnoxyd ≙ 0,5 Mol-% zu und erwärmt bis auf 180°C, wobei sich Methanol abspaltet. Man hält die Temperatur während 6 Stunden bei 180°C.

Mittels Säulenchromatographie wird das erhaltene braune Oel nachgereinigt, wobei 72,4 % der eingesetzten Menge in Form eines viskosen Oeles erhalten werden.

| | | | |
|---|---|---|---|
| Analyse: berechnet | 71,60 % C | 9,31 % H | 4,78 % S |
| gefunden | 71,66 % C | 9,08 % H | 4,90 % S |

6. Herstellung von 3-[3-tert.Butyl-4-hydroxyphenyl]-2-methylpropansäure methylester

190 g 3-[3,5-di-tert-Butyl-4-hydroxyphenyl]-2-methylpropansäuremethylester (0,62 mol) werden mit 11,40 g Campher-(10)-sulfonsäure (0,05 mol) 24 Stunden bei 120° C gerührt. Die Reaktion wird mittels GC kontrolliert. Das Reaktionsgemisch wurde in Essigester aufgenommen, mit Natriumhydrogencarbonat und Wasser neutral gewaschen, getrocknet und das Lösungsmittel eingeengt. Das Rohprodukt wird am Hochvakuum destilliert. Kp 115° C/15$^2$mm[Hg].

Es werden 93 g 3-[3-tert.Butyl-4-hydroxyphenyl]-2-methylpropansäuremethylester als weisser Feststoff erhalten. Smp.: 49° C

Formel: 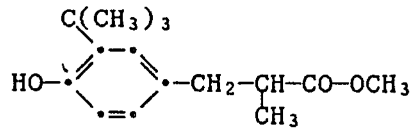

7. Herstellung von 3-[3-tert.Butyl-4-hydroxyphenyl]-2-methylpropansäure-2-methylpentylester

2,50 g 3-[3-tert.Butyl-4-hydroxyphenyl]-2-methylpropansäuremethylester (0,01 mol) werden mit 7,80 g 2-Methyl-1-pentanol (0,076 mol) und 0,10 g Dibutylzinnoxyd (0,00040 mol) 24 Stunden bei 120° C gerührt. Das bei der Reaktion gebildete Methanol wird während der Reaktion laufend abdestilliert. Die Reaktion wird mittels DC kontrolliert. Das überschüssige 2-Methyl-1-pentanol wird bei 100° C und 15 mm [Hg] abdestilliert und das Rohprodukt über Kieselgel chromatografiert. Es werden 1,60 g 3-[3-tert.-Butyl-4-hydroxyphenyl]-2-methylpropansäure-2-methylpentylester als farbloses viskoses Oel erhalten.

8. Herstellung von 3-[3-tert.Butyl-4-hydroxyphenyl]-2-methylpropansäure-9-octadecenylester

In Beispiel 6 analoger Herstellungsweise wird aus 47,3 g 3-[3-tert.-Butyl-4-hydroxyphenyl]-2-methylpropansäuremethylester (0,20 mol), 112 g 9-Octadecen-1-ol (0,40 mol) und 0,50 g Dibutylzinnoxyd (0,0020 mol) 73 g 3-[3-tert.Butyl-4-hydroxyphenyl]-2-methylpropansäure-9-octadecenylester als farbloses Oel erhalten.

9.-11. Prüfung der Stabilisatoren

0,087 g des Gelbkupplers der Formel

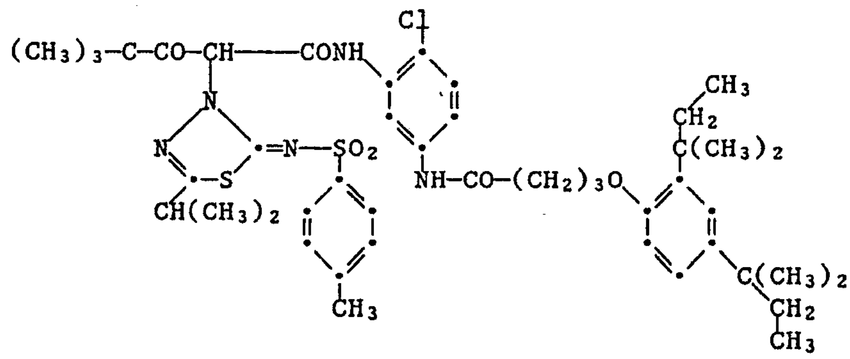

und 0,029 g eines der in der nachfolgenden Tabelle angegebenen Lichtschutzmittel werden in 2 ml eines Gemisches von Dibutylphthalat/Aethylacetat (1,5 g/100 ml) gelöst. Zu 1,0 ml dieser Lösung gibt man 9,0 ml einer 2,3 %igen wässrigen Gelatinelösung, die auf einen pH-Wert von 6,5 eingestellt ist und 1,744 g/lt Netzmittel ®Nekal BX (Diisobutylnaphthalin-sulfonsäure-Na-salz) enthält.

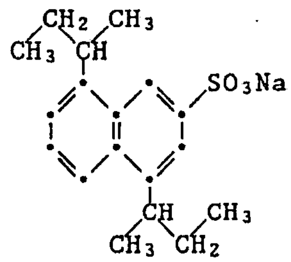

Danach emulgiert man mit Ultraschall 3 Minuten.

Zu 5,0 ml der so erhaltenen Kuppleremulsion gibt man 2 ml einer Silberbromid-Emulsion mit einem Silbergehalt von 6 g pro Liter, 1,0 ml einer 0,7 %igen wässrigen Lösung des Härters der Formel:

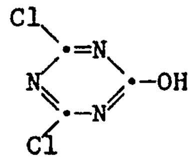

und vergiesst es auf ein 13x18 cm kunststoffbeschichtetes Papier. Nach einer Härtungszeit von 7 Tagen werden die Proben durch einen Silber-Stufenkeil mit 125 Lux•s belichtet und anschliessend im ®Extaprint 2-Prozess der Firma Kodak entwickelt.

Die so erhaltenen Farbstufenkeile werden in einem Atlas Weather-Ometer hinter einem UV-Filter (Kodak 2C) mit einer 2500 W Xenonlampe mit total 60 Kilojoule per $cm^2$ bestrahlt.

In der folgenden Tabelle sind die prozentualen Farbdichteabnahmen bei einer ursprünglichen Farbdichte von 1,0 enthalten.

Tabelle:

| Bsp. | Stabilisator | Verbindung gemäss Beispiel | 60 kJ/cm² (UV-Filter)<br>Prozentuale Farbdichteabnahme |
|---|---|---|---|
| Vgl. | Keiner | - | 33 |
| 9 | 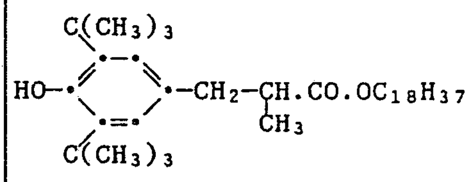 | 2 | 16 |
| 10 | 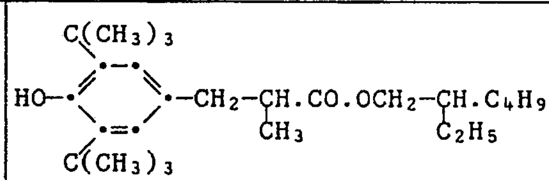 | 3 | 25 |
| 11 | 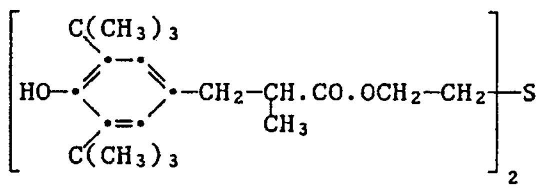 | 4 | 12 |

## Ansprüche

1. Farbphotographisches Aufzeichnungsmaterial, enthaltend in mindestens einer der chromogenen farbphotographischen Schichten als Stabilisator mindestens eine Verbindung der Formel I,

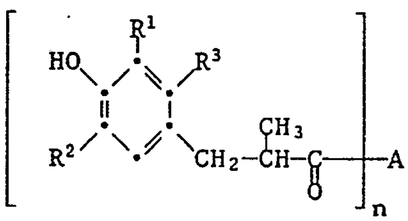

(I),

wobei

$R^1$ = Alkyl mit 1 bis 18 C-Atomen, Cycloalkyl mit 5 bis 12 C-Atomen, mit 1 bis 3 $C_1$-$C_4$-Alkylgruppen substituiertes Cycloalkyl mit 5 bis 12 Ring-C-Atomen, Phenyl oder Aralkyl mit 7 bis 9 C-Atomen,

$R^2$ = -H, Alkyl mit 1 bis 18 C-Atomen, Cycloalkyl mit 5 bis 12 C-Atomen, mit 1 bis 3 $C_1$-$C_4$-Alkylgruppen substituiertes Cycloalkyl mit 5 bis 12 Ring-C-Atomen, Phenyl oder Aralkyl mit 7 bis 9 C-Atomen und

$R^3$ = -H oder $CH_3$ bedeuten und

n die Zahl 1 oder 2 bedeutet, wobei,

wenn n = 1 ist,

A = $-OR^4$ oder

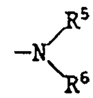

ist, und
$R^4$ die Bedeutung von Alkyl mit 1 bis 24 C-Atomen, durch wenigstens ein -O-, -S- oder $-NR^7-$ unterbrochenes $C_3$-$C_{20}$-Alkyl oder durch wenigstens ein -O-, -S- oder $-NR^7-$ unterbrochenes $C_3$-$C_{20}$-Alkyl, das mit wenigstens einer Hydroxylgruppe substituiert ist, hat, oder eine Gruppe der Formel

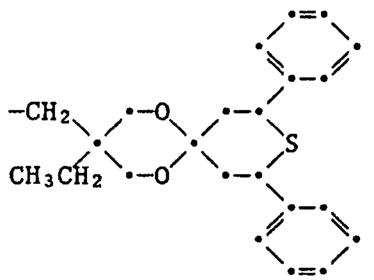

oder

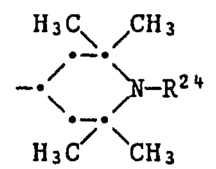

ist,
wobei
$R^7$ Wasserstoff, $C_1$-$C_{12}$-Alkyl oder Phenyl und $R^{24}$ Alkyl mit 1 bis 4 Kohlenstoffatomen, Allyl, Benzyl ein Rest der Formel

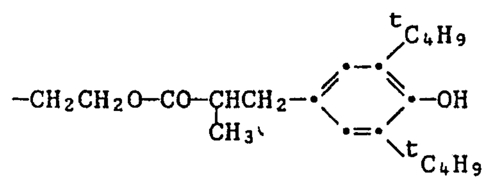

oder $COR^{25}$ ist,
worin $R^{25}$ Alkyl mit 1 bis 24 Kohlenstoffatomen oder Allyl ist, oder $R^4$ die Bedeutung von durch -OH, $-OCOR^8$ oder $-P(O)(OR^9)_2$ substituiertem $C_1$-$C_{24}$-Alkyl hat, wobei
$R^8$ $C_1$-$C_{18}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl oder $C_7$-$C_{15}$-Alkaryl und
$R^9$ Wasserstoff, $C_{1-18}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl, Naphthyl oder $C_7$-$C_{15}$-Alkaryl bedeuten, oder
$R^4$ die Bedeutung von $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, $C_7$-$C_{18}$-Alkaryl, Phenyl, einer Gruppe $-CH_2CH(OH)R^{10}$, wobei $R^{10}$ $C_7$-$C_{12}$-Aralkyl oder $-CH_2OR^{11}$ und $R^{11}$ Cyclohexyl, Phenyl, Tolyl oder Benzyl bedeuten, hat, oder
$R^4$ die Bedeutung einer Gruppe der Formel

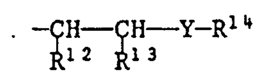

hat, wobei
$R^{12}$ und $R^{13}$, unabhängig voneinander, Wasserstoff oder $C_1$-$C_{18}$-Alkyl,
Y -O- oder -S- und
$R^{14}$ eine Gruppe der Formel

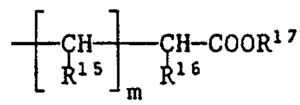

darstellen, wobei
$R^{15}$ und $R^{16}$, unabhängig voneinander, Wasserstoff oder Methyl, m 0 oder 1 und
$R^{17}$ Wasserstoff, $C_1$-$C_{24}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl oder $C_7$-$C_{16}$-Alkaryl darstellen, oder
$R^{14}$ eine Gruppe der Formel

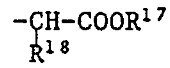

darstellt, wobei $R^{18}$ Wasserstoff, $C_1$-$C_{18}$-Alkyl oder eine Gruppe -$COOR^{17}$, wobei $R^{17}$ genannte Bedeutung hat, darstellt, oder
$R^{14}$ Wasserstoff, Phenyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, Benzyl oder eine Gruppe der Formel

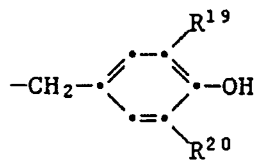

darstellt, wobei
$R^{19}$ und $R^{20}$, unabhängig voneinander, -H, Methyl oder -$C(CH_3)_3$ bedeuten, mit der Massgabe, dass $R^{19}$ und $R^{20}$ nicht gleichzeitig -H sind, und
$R^5$ und $R^6$, unabhängig voneinander, Wasserstoff, $C_1$-$C_{12}$-Alkyl, durch mindestens ein -O- unterbrochenes $C_3$-$C_{20}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, Phenyl, durch $C_1$-$C_{12}$-Alkyl, $C_1$-$C_4$-Alkoxy oder Halogen substituiertes Phenyl, $C_3$-$C_8$-Alkenyl, $C_7$-$C_{15}$-Aralkyl oder $C_2$-$C_4$-Hydroxyalkyl darstellen, oder
$R^5$ und $R^6$ zusammen Tetramethylen oder Pentamethylen oder durch -O-, -$NR^{21}$-oder -$S(O)_q$- unterbrochenes Tetramethylen oder Pentamethylen darstellen, wobei $R^{21}$ Wasserstoff, $C_1$-$C_{12}$-Alkyl oder Phenyl bedeutet und q = 0, 1 oder 2 ist, oder,
wenn n = 2 ist,
A eine Gruppe der Formel -O-X'-O- bedeutet, wobei
X' = $C_2$-$C_{10}$-Alkylen, $C_4$-$C_8$-Alkenylen, $C_4$-$C_8$-Alkinylen, Cyclohexylen, durch ein oder mehrere Glieder der Reihe

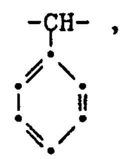

-O-, -S(O)q- und -$NR^{22}$-unterbrochenes $C_4$-$C_{40}$-Alkylen, wobei $R^{22}$ Wasserstoff oder $C_1$-$C_{12}$-Alkyl oder Phenyl und q = 0, 1 oder 2 sind, bedeutet, oder
A eine Gruppe der Formel

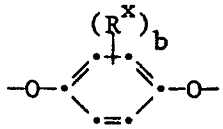

bedeutet,
wobei $R^x$ eine Alkylgruppe mit 1 bis 8 C-Atomen darstellt und b = 0, 1 oder 2 ist, oder
A eine Gruppe der Formel

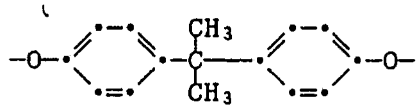

bedeutet, oder
A eine Gruppe der Formel

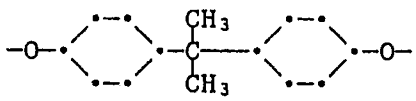

bedeutet, oder
A eine Gruppe der Formel
$-O-CH_2-CH(OH)-CH_2-O-X^2-O-CH_2-CH(OH)-CH_2-O-$ bedeutet,
wobei $X^2 = X'$ entspricht oder

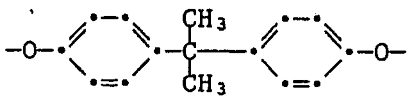

oder

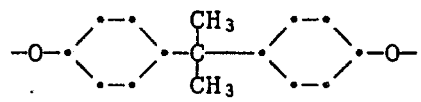

ist, oder
A eine Gruppe der Formel

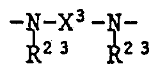

,

wobei
$X^3$ $C_2$-$C_{10}$-Alkylen und $R^{23}$ Wasserstoff, $C_1$-$C_{12}$-Alkyl oder Phenyl sind, bedeutet.

2. Farbphotographisches Aufzeichnungsmaterial gemäss Anspruch 1, enthaltend mindestens eine Verbindung der Formel I, wobei
$R^1$ = Alkyl mit 1 bis 8 C-Atomen, Cyclohexyl, Phenyl oder $C_7$-$C_9$-Phenylalkyl,
$R^2$ = Wasserstoff, Alkyl mit 1 bis 8 C-Atomen, Cyclohexyl, Phenyl oder $C_7$-$C_9$-Phenylalkyl, und
$R^3$ = Wasserstoff bedeuten.

3. Farbphotographisches Aufzeichnungsmaterial gemäss Anspruch 1, enthaltend mindestens eine Verbindung der Formel I, wobei,
wenn n = 1 ist,
A die Bedeutung von $-OR^4$ oder $-NR^5R^6$ hat, wobei
$R^4$ die Bedeutung von Alkyl mit 1 bis 18 C-Atomen, durch 1 bis 3 -O- oder -S- unterbrochenes $C_3$-$C_{20}$-Alkyl oder durch 1 bis 3 -O- oder -S- unterbrochenes $C_3$-$C_{20}$-Alkyl, das mit 1 bis 3 -OH substituiert ist, oder $R^4$ die Bedeutung von durch $-P(O)(OR^9)_2$ substituiertem $C_1$-$C_9$-Alkyl hat, wobei $R^9$ Wasserstoff, $C_1$-$C_{12}$-Alkyl, $C_5$-$C_{12}$-Cycloalkyl, $C_3$-$C_{18}$-Alkenyl, Benzyl, Phenyl oder Tolyl darstellt, oder
$R^4$ die Bedeutung von $C_5$-$C_{12}$-Cycloalkyl, $C_2$-$C_{18}$-Alkenyl, $C_7$-$C_{10}$-Aralkyl, $C_7$-$C_{18}$-Alkaryl, Phenyl oder einer Gruppe $-CH_2CH(OH)R^{10}$ hat, wobei $R^{10}$ $C_7$-$C_{10}$-Aralkyl oder $-CH_2OR^{11}$ bedeutet, mit der Bedeutung für $R^{11}$ von Cyclohexyl, Phenyl, Tolyl oder Benzyl, oder
$R^4$ eine Gruppe der Formel

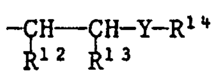

bedeutet, wobei
$R^{12}$ und $R^{13}$, unabhängig voneinander, Wasserstoff oder $C_1$-$C_{12}$-Alkyl bedeuten,
Y = -O- oder -S- ist, und
$R^{14}$ eine Gruppe der Formel

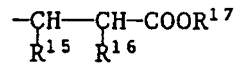

ist,
wobei
$R^{15}$ und $R^{16}$, unabhängig voneinander, Wasserstoff oder Methyl und
$R^{17}$ $C_1$-$C_{12}$-Alkyl, Cyclohexyl, $C_2$-$C_{18}$-Alkenyl, $C_7$-$C_9$-Aralkyl, Phenyl oder $C_7$-$C_{16}$-Alkaryl sind, oder
$R^{14}$ eine Gruppe der Formel

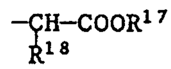

bedeutet, wobei
$R^{18}$ Wasserstoff, $C_1$-$C_{12}$-Alkyl oder eine Gruppe -$COOR^{17}$ darstellt und wobei $R^{17}$ die obengenannte Bedeutung hat, oder
$R^{14}$ Phenyl, Cyclohexyl, $C_2$-$C_9$-Alkenyl, Benzyl oder eine Gruppe der Formel

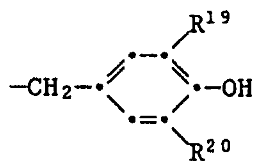

darstellt,
wobei
$R^{19}$ und $R^{20}$, unabhängig voneinander, -H, Methyl oder -$C(CH_3)_3$ darstellen, mit der Massgabe, dass $R^{19}$ und $R^{20}$ nicht gleichzeitig -H sind, und
$R^5$ und $R^6$, unabhängig voneinander, Wasserstoff, $C_1$-$C_{12}$-Alkyl, durch ein bis drei -O- unterbrochenes $C_3$-$C_{20}$-Alkyl, Cyclohexyl, Phenyl, $C_3$-$C_6$-Alkenyl oder Benzyl oder
$R^5$ und $R^6$ zusammen Tetramethylen oder Pentamethylen oder durch ein -O-unterbrochenes Tetramethylen darstellen, oder,
wenn n = 2 ist,
A eine Gruppe der Formel -O-X′-O- darstellt, wobei
X′ die Bedeutung $C_2$-$C_8$-Alkylen, $C_4$-$C_8$-Alkenylen, $C_4$-$C_{12}$-Alkylen, das durch 1 bis 3 Glieder der Reihe

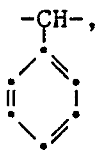

-O-, -S(O)q- und -$NR^{22}$- unterbrochen ist, wobei $R^{22}$ die Bedeutung von Wasserstoff, $C_1$-$C_{12}$-Alkyl oder Phenyl hat und q = 0, 1 oder 2 ist; Phenylen,
-$CH_2$-C≡C-$CH_2$- oder -$(CH_2CH_2O)_aCH_2CH_2$-, wobei
a = 1, 2 oder 3 ist, hat oder A die Bedeutung einer Gruppe der Formel

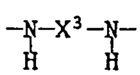

hat, wobei $X^3$ Alkylen mit 4 bis 8 C-Atomen ist.

4. Farbphotographisches Aufzeichnungsmaterial gemäss Anspruch 1, enthaltend mindestens eine Verbindung der Formel I, wobei $R^1$ Alkyl mit 1 bis 4 C-Atomen, $R^2$ Wasserstoff oder Alkyl mit 1 bis 4 C-Atomen und $R^3$ Wasserstoff bedeuten.

5. Farbphotographisches Auzeichnungsmaterial gemäss Anspruch 1, enthaltend mindestens eine Verbindung der Formel I, wobei,

wenn n = 1 ist,
A die Bedeutung von $-OR^4$ hat, wobei
$R^4$ Alkyl mit 1 bis 18 C-Atomen, Alkenyl mit 2 bis 18 C-Atomen oder durch ein oder zwei -O-unterbrochenes Alkyl mit 3 bis 12 C-Atomen bedeutet, oder $R^4$ eine Gruppe der Formel $-CH_2-CH_2-S-(CH_2)_m-CH_2-COOR^{17}$ bedeutet, wobei
m 0 oder 1 und $R^{17}$ $C_1$-$C_4$-Alkyl sind, oder
$R^4$ $-CH_2-P(O)(OR^9)_2$ oder $-CH_2-CH_2-P(O)(OR^9)_z$ bedeutet, wobei
$R^9$ $C_2$-$C_5$-Alkyl ist, oder
A die Bedeutung von

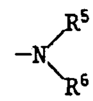

hat,
wobei
$R^5$ = Wasserstoff, $C_1$-$C_8$-Alkyl, $C_2$-$C_4$-Alkenyl oder durch ein -O-unterbrochenes $C_3$-$C_8$-Alkyl ist, und
$R^6$ = $C_1$-$C_8$-Alkyl, $C_2$-$C_4$-Alkenyl oder durch ein -O- unterbrochenes $C_3$-$C_8$-Alkyl ist, oder
wenn n = 2 ist,
A die Bedeutung einer Gruppe der Formel $-O-X'-O-$ hat, wobei
$X'$ eine Alkylengruppe mit 2 bis 4 C-Atomen, $-CH_2-CH=CH-CH_2-$, eine Alkenylengruppe mit 4 bis 6 C-Atomen oder eine durch ein -S-, -O-, oder

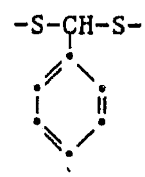

unterbrochene Alkylengruppe mit 2 bis 6 C-Atomen ist, oder
A die Bedeutung einer Gruppe der Formel

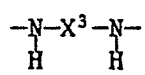

hat, wobei $X^3$ Alkylen mit 4 bis 8 C-Atomen ist.

6. Farbphotographisches Aufzeichnungsmaterial gemäss Anspruch 1, enthaltend mindestens eine Verbindung der Formel I, worin $R^1$ und $R^2$ eine tert.-Butylgruppe und $R^3$ Wasserstoff bedeuten.

7. Farbphotographisches Aufzeichnungsmaterial gemäss Anspruch 1, enthaltend mindestens eine Verbindung der Formel I, worin,
wenn n = 1 ist,
A die Bedeutung von $-OR^4$ hat, wobei
$R^4$ Alkyl mit 1-18 C-Atomen, Alkenyl mit 3 bis 18 C-Atomen, $-CH_2-CH_2-S-C(CH_3)_3$ oder $-CH_2-CH_2-O-CH_2-CH_2-O-C_2H_5$ bedeutet, oder,
wenn n = 2 ist,
A die Bedeutung von $-O-X'-O-$ hat, wobei $X'$ Alkylen mit 2 bis 4 C-Atomen oder durch ein -O- oder -S-unterbrochenes Alkylen mit 4 bis 6 C-Atomen ist.

8. Farbphotographisches Aufzeichnungsmaterial gemäss Anspruch 1, enthaltend mindestens eine Verbindung der Formel I, worin
$R^1$ und $R^2$ jeweils tert.-Butyl und $R^3$ Wasserstoff bedeuten, und, wenn n=1 ist, A $-OR^4$ ist, wobei $R^4$ die Bedeutung von $-CH_3$,

$-CH_3$, 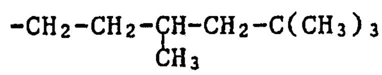

oder $C_{18}H_{37}$ hat oder wenn n = 2 ist,
A die Bedeutung von -O-X'-O- hat, wobei X' = $-CH_2-CH_2-S-CH_2-CH_2-$ ist.

9. Farbphotographisches Aufzeichnungsmaterial gemäss Anspruch 1, enthaltend mindestens eine Verbindung der Formel I, worin $R^1$ tert.-Butyl, $R^2$ und $R^3$ Wasserstoff darstellen, und, wenn n = 1 ist, A eine Gruppe $-OR^4$ darstellt, wobei $R^4$ Methyl, $-C_8H_{17}$ oder $-(CH_2)_8CH=CH(CH_2)_7CH_3$ ist oder, wenn n = 2 ist, A eine Gruppe der Formel -O-X'-O- darstellt und dabei X' $-CH_2-CH_2-S-CH_2-CH_2-$ oder $-CH_2-CH_2-O-CH_2-CH_2-$ ist.

10. Verbindungen der Formel Ia,

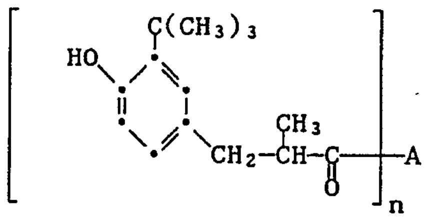 ,

Ia

worin n eine Zahl von 1 oder 2 bedeutet und,
wenn n = 1 ist,
A = $-OR^4$ oder

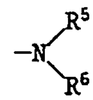

ist, und
$R^4$ die Bedeutung von -H, Alkyl mit 1 bis 24 C-Atomen, durch wenigstens ein -O-, -S- oder $-NR^7-$ unterbrochenes $C_3$-$C_{20}$-Alkyl oder durch wenigstens ein -O-, -S- oder $-NR^7-$ unterbrochenes $C_3$-$C_{20}$-Alkyl, das mit wenigstens einer Hydroxygruppe substituiert ist, hat, oder eine Gruppe der Formel

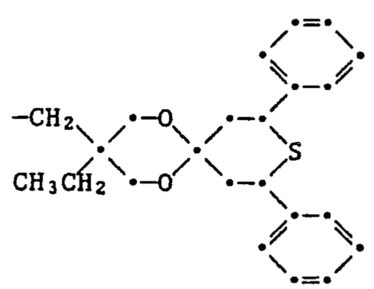 oder 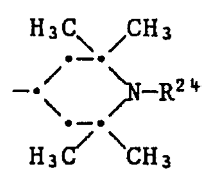

ist,
wobei
$R^7$ Wasserstoff, $C_1$-$C_{12}$-Alkyl oder Phenyl und $R^{24}$ Alkyl mit 1 bis 4 Kohlenstoffatomen, Allyl, Benzyl ein Rest der Formel

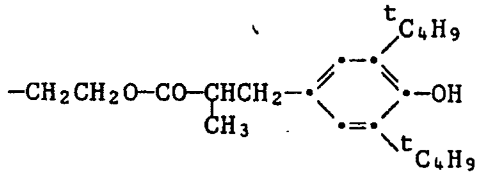

oder $COR^{25}$ ist,
worin $R^{25}$ Alkyl mit 1 bis 24 Kohlenstoffatomen oder Allyl ist, oder
$R^4$ die Bedeutung von durch -OH, $-OCOR^8$ oder $-P(O)(OR^9)_2$ substituiertem $C_1$-$C_{24}$-Alkyl, hat, wobei
$R^8$ $C_1$-$C_{18}$-Alkyl, $C_5$-$C_{12}$-Cycloalkyl, mit 1 bis 3 $C_1$-$C_4$-Alkylgruppen substituiertes Cycloalkyl mit 5 bis 12

Ring-C-Atomen, $C_2$-$C_{18}$-Alkenyl, $C_7$-$C_{15}$-Aralkyl, Phenyl, Naphthyl, oder $C_7$-$C_{15}$-Alkaryl und $R^9$ Wasserstoff, $C_{1-18}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, $C_7$-$C_{15}$-Aralkyl, Phenyl, Naphthyl oder $C_7$-$C_{15}$-Alkaryl bedeuten, oder

$R^4$ die Bedeutung von $C_5$-$C_{12}$-Cycloalkyl, mit 1 bis 3 $C_1$-$C_4$-Alkylgruppen substituiertem Cycloalkyl mit 5 bis 12 Ring-C-Atomen, $C_2$-$C_{18}$-Alkenyl, $C_7$-$C_{15}$-Aralkyl, $C_7$-$C_{18}$-Alkaryl, Phenyl, Naphthyl, einer Gruppe $-CH_2CH(OH)R^{10}$, wobei $R^{10}$ $C_7$-$C_{12}$-Aralkyl oder $-CH_2OR^{11}$ und $R^{11}$ Cyclohexyl, Phenyl, Tolyl oder Benzyl bedeuten, hat, oder

$R^4$ die Bedeutung einer Gruppe der Formel

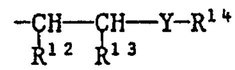

hat, wobei

$R^{12}$ und $R^{13}$, unabhängig voneinander, Wasserstoff oder $C_1$-$C_{18}$-Alkyl,

Y -O- oder -S- und

$R^{14}$ eine Gruppe der Formel

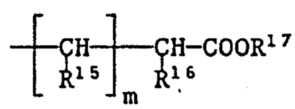

darstellen, wobei

$R^{15}$ und $R^{16}$, unabhängig voneinander, Wasserstoff oder Methyl, m 0 oder 1 und

$R^{17}$ Wasserstoff, $C_1$-$C_{24}$-Alkyl, mit 1 bis 3 $C_1$-$C_4$-Alkylgruppen substituiertes Cycloalkyl mit 5 bis 12 Ring-C-Atomen, $C_5$-$C_{12}$-Cycloalkyl, $C_2$-$C_{18}$-Alkenyl, $C_7$-$C_{15}$-Aralkyl, Phenyl oder $C_7$-$C_{16}$-Alkaryl darstellt, oder $R^{14}$ eine Gruppe der Formel

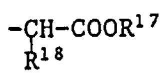

darstellt, wobei $R^{19}$ Wasserstoff, $C_1$-$C_{18}$-Alkyl oder eine Gruppe $-COOR^{17}$, wobei $R^{17}$ genannte Bedeutung hat, darstellt, oder

$R^{14}$ Wasserstoff, Phenyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, $C_2$-$C_{18}$-Alkenyl, Benzyl oder eine Gruppe der Formel

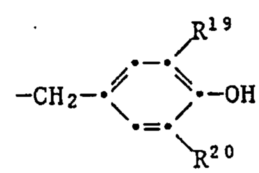

darstellt, wobei

$R^{19}$ und $R^{20}$, unabhängig voneinander, -H, Methyl oder $-C(CH_3)_3$ bedeuten, mit der Massgabe, dass $R^{19}$ und $R^{20}$ nicht gleichzeitig -H sind, und

$R^5$ und $R^6$, unabhängig voneinander, Wasserstoff, $C_1$-$C_{12}$-Alkyl, durch mindestens ein -O- unterbrochenes $C_3$-$C_{20}$-Alkyl, $C_5$-$C_{12}$-Cycloalkyl, mit 1 bis 3 $C_1$-$C_4$-Alkylgruppen substituiertes Cycloalkyl mit 5 bis 12 Ring-C-Atomen, Phenyl, durch $C_1$-$C_{12}$-Alkyl, $C_1$-$C_4$-Alkoxy oder Halogen substituiertes Phenyl, $C_3$-$C_8$-Alkenyl, $C_7$-$C_{15}$-Aralkyl oder $C_2$-$C_4$-Hydroxyalkyl darstellen oder

$R^5$ und $R^6$ zusammen Tetramethylen oder Pentamethylen oder durch -O-, $-NR^{21}$-oder $S(O)_q$- unterbrochenes Tetramethylen oder Pentamethylen darstellen, wobei $R^{21}$ Wasserstoff, $C_1$-$C_{12}$-Alkyl oder Phenyl bedeutet und

q = 0, 1 oder 2 ist, oder, wenn $n'$ = 2 ist,

A eine Gruppe der Formel $-O-X'-O-$ bedeutet, wobei

$X'$ = $C_2$-$C_{10}$-Alkylen, $C_4$-$C_8$-Alkenylen, $C_4$-$C_8$-Alkinylen, Cyclohexylen, durch ein oder mehrere Glieder aus

der Reihe

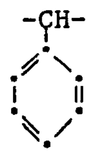 ,

-O-, -S(O)q- und -$NR^{22}$- unterbrochenes $C_4$-$C_{40}$-Alkylen, wobei $R^{22}$ Wasserstoff oder $C_1$-$C_{12}$-Alkyl oder Phenyl, und q = 0, 1 oder 2 sind, bedeutet, oder
A eine Gruppe der Formel

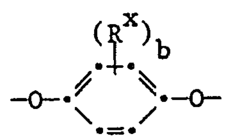

ist, wobei $R^x$ eine Alkylgruppe mit 1 bis 8 C-Atomen darstellt und b = 0, 1 oder 2 ist, oder
A eine Gruppe der Formel

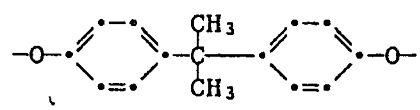

bedeutet, oder
A eine Gruppe der Formel

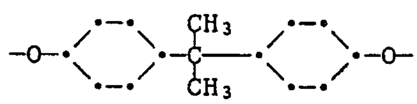

bedeutet, oder
A eine Gruppe der Formel
-O-$CH_2$-CH(OH)-$CH_2$-O-$X^2$-O-$CH_2$-CH(OH)-$CH_2$-O- bedeutet,
wobei $X^2$ = $X'$ entspricht oder

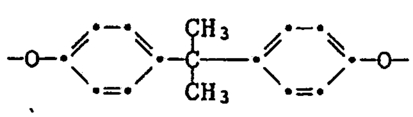 oder 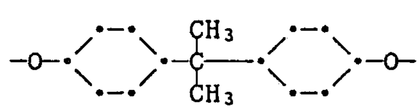

ist, oder
A eine Gruppe der Formel

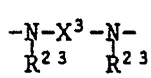 ,

wobei
$X^3$ $C_2$-$C_{10}$-Alkylen und $R^{23}$ Wasserstoff, $C_1$-$C_{12}$-Alkyl oder Phenyl sind, bedeutet.
11. Verbindungen der Formel Ia gemäss Anspruch 10, worin n = 1 ist, und A die Bedeutung von -$OR^4$ hat und
$R^4$ eine Alkylgruppe mit 1 bis 18 C-Atomen oder eine Alkenylgruppe mit 2 bis 18 C-Atomen darstellt, oder
n = 2 ist und
A die Bedeutung von -O-$X'$-O- hat, wobei $X'$ = $C_2$-$C_8$-Alkylen oder $C_4$-$C_{12}$-Alkylen, das durch 1 bis 3 Gruppen der Reihe -O-, -S- oder -$NR^{22}$-unterbrochen ist, wobei $R^{22}$ die Bedeutung von Wasserstoff, $C_1$-$C_{12}$-

Alkyl oder Phenyl hat, oder Phenylen darstellt.

12. Verbindungen der Formel Ia gemäss Anspruch 10, worin $n = 1$ ist, und $R^4$ die Bedeutung von $C_1$ bis $C_{18}$-Alkyl oder $C_{12}$-$C_{18}$-Alkenyl hat, oder worin $n = 2$ ist, und A die Bedeutung von $-O-CH_2-CH_2-S-CH_2-CH_2-O-$ oder $-O-CH_2-CH_2-O-CH_2-CH_2-O-$ hat.

13. Verwendung von Verbindungen der Formel I nach Anspruch 1 als Stabilisatoren und/oder als Kupplerlösungsmittel für farbphotographisches Aufzeichnungsmaterial.

14. Verwendung von Verbindungen der Formel Ia nach Anspruch 7 als Stabilisatoren und/oder als Kupplerlösungsmittel für farbphotographisches Aufzeichnungsmaterial.